# FASCICULE DE BREVET EUROPEEN

(11) **EP 1 299 416 B1**
(45) Date de publication et mention de la délivrance du brevet: **12.04.2006**
(21) Numéro de dépôt: 01984240.0
(22) Date de dépôt: 05.07.2001
(51) Int. Cl.: C07K 14/435, C12N 15/12, C12N 15/82, A61K 38/17, A61P 31/00

(54) **PEPTIDES ANTIFONGIQUES ET/OU ANTIBACTERIENS, LEURS PREPARATIONS ET LES COMPOSITIONS LES CONTENANT**
PEPTIDE MIT ANTIFUNGISCHER UND/ODER ANTIBAKTERIELLER WIRKUNG, IHRE HERSTELLUNG UND DIESE ENTHALTENDE ZUSAMMENSETZUNGEN
ANTIFUNGAL AND/OR ANTIBACTERIAL PEPTIDES, PREPARATION METHODS AND COMPOSITION CONTAINING SAME

(30) Priorité: 13.07.2000 FR 0009248; 19.09.2000 FR 0011949
(43) Date de publication de la demande: 09.04.2003
(73) Titulaire: Entomed, 67400 Illkirch (FR)
(72) Inventeur: DIMARCQ, Jean-Luc, F-67000 Strasbourg (FR); LEGRAIN, Michèle, F-67140 Stotzheim (FR); MENIN, Laure, CH-1053 Cugy (VD) (CH)
(74) Mandataire: Breese, Pierre
(86) Numéro de dépôt international: PCT/FR2001/002164
(87) Numéro de publication internationale: WO 2002/006324

(56) Documents cités:
- WO-A-99/53053
- FR-A- 2 695 392
- LAMBERTY M ET AL: "Insect immunity - Isolation from the lepidopteran Heliothis virescens of a novel insect defensin with potent antifungal activity" JOURNAL OF BIOLOGICAL CHEMISTRY,US,AMERICAN SOCIETY OF BIOLOGICAL CHEMISTS, BALTIMORE, MD, vol. 274, no. 14, 2 avril 1999 (1999-04-02), pages 9320-9326, XP002112857 ISSN: 0021-9258
- MARTIN E ET AL: "DEFENSINS AND OTHER ENDOGENOUS PEPTIDE ANTIBIOTICS OF VERTEBRATES" JOURNAL OF LEUKOCYTE BIOLOGY,FEDERATION OF AMERICAN SOCIETIES FOR EXPERIMENTAL,US, vol. 58, août 1995 (1995-08), pages 128-136, XP000972936 ISSN: 0741-5400
- CHUNG KYUNG T ET AL: "Viresin: A novel antibacterial protein from immune hemolymph of Heliothis virescens pupae." EUROPEAN JOURNAL OF BIOCHEMISTRY, vol. 267, no. 3, février 2000 (2000-02), pages 677-683, XP002167332 ISSN: 0014-2956

## Description

La présente invention a pour objet de nouveaux peptides ayant des propriétés antibactériennes et antifongiques. L'invention concerne également la préparation de ces peptides et les compositions les contenant utilisables en agriculture et en thérapie humaine ou animale.

On a décrit dans l'art antérieur de nombreuses substances d'origine naturelle, notamment des peptides présentant des propriétés anti-microbiennes, plus particulièrement bactéricides ou fongicides. De tels peptides sont utiles pour le traitement des maladies fongiques tant chez les plantes que chez l'homme (De Lucca *et al.,* 1999, Antimicrob. Agents Chemother. 43, 1-11). En santé humaine, on peut rappeler que l'incidence des infections fongiques opportunistes a connu une progression très nette au cours des dernières années. Les mycoses invasives sont des infections très graves qui sont provoquées par des champignons répandus dans la nature et qui deviennent pathogènes chez des sujets immunodéprimés. L'immunosuppression peut être le résultat de différentes causes : corticothérapie, chimiothérapie, transplantations, infection par le VIH. Les infections fongiques opportunistes représentent à l'heure actuelle une cause importante de mortalité chez l'homme. Elles peuvent être causées par des levures, principalement du genre *Candida,* ou des champignons filamenteux, principalement du genre *Aspergillus*. Chez les patients immunodéprimés, il est fréquent d'observer l'échec des traitements antifongiques en raison de la toxicité de ceux-ci, par exemple le traitement par l'amphotéricine B, ou de l'apparition de champignons résistants, par exemple la résistance de *Candida albicans* aux dérivés azotés. Il est donc essentiel de développer de nouveaux médicaments anti-fongiques dérivés de molécules innovantes.

La production de peptides antimicrobiens représente, chez une grande variété d'espèces animales et végétales, un mécanisme essentiel de défense immunitaire contre les infections. En particulier, les insectes présentent une résistance très efficace contre les bactéries et les champignons. Cette réponse repose pour une large part sur la synthèse rapide de plusieurs familles de peptides antimicrobiens à large spectre d'activité (Bulet *et al*. (1999) *Dev. Comp. Immunol.* 23, 329-344). Cette synthèse est induite par une blessure septique ou par l'injection d'une faible dose de bactéries (Hoffmann *et al*. (1999) *Science* 284, 1313-1318). A ce jour, les peptides antimicrobiens d'insectes ont été surtout caractérisés à partir d'insectes ayant une métamorphose complète pendant le développement, par exemple les diptères, lépidoptères et coléoptères. Parmi les peptides anti-microbiens induits chez ces insectes, on peut distinguer les quatre groupes suivants :
- Des peptides cationiques de 4 kDa, formant deux hélices α amphipathiques. Dans ce groupe, on classe en particulier les cécropines.
- Des peptides cationiques riches en proline, de taille comprise entre 2 kDa et 4 kDa qui peuvent être glycosylés, comme par exemple, la drosocine, la pyrrhocoricine, et les lébocines, ou non glycosylés comme par exemple, les apidaecines et les métalnikowines.
- Plusieurs polypeptides distincts, ayant un poids moléculaires de 8 à 27 kDa, pour la plupart cationiques et fréquemment riches en résidus glycine comme par exemple les attacines, les sarcotoxines II, les diptéricines et la coléoptéricine.
- Des peptides comprenant des ponts disulfures intramoléculaires. Dans ce groupe, on classe les défensines d'insecte (4 kDa, 3 ponts disulfure), la drosomycine (4 kDa, 4 ponts disulfure) et la thanatine (2 kDa, 1 pont disulfure).

Parmi ceux-ci, la présente invention s'intéresse à des peptides présentant une structure tridimensionnelle du type comportant une hélice α et un feuillet β antiparallèle reliés par trois ponts disulfures, également désignée structure CSαβ. Ces peptides présentent une activité antifongique utile dans le traitement d'infection chez l'homme et l'animal ainsi que chez les plantes. L'invention concerne tout particulièrement l'héliomicine qui est un peptide isolé à partir de l'hémolymphe de lépidoptère *Heliothis virescens.* La séquence et les propriétés de l'héliomicine sont décrites dans la demande de brevet internationale PCT publiée sous le No. WO 9953053.

Dans les séquences peptidiques rapportées ci-après, les acides aminés sont représentés par leur code à une lettre, mais ils peuvent être aussi représentés par leur code à trois lettres selon la nomenclature ci-dessous.

| | | |
|---|---|---|
| A | Ala | alanine |
| C | Cys | cystéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | sérine |
| T | Thr | thréonine |
| V | Val | valine |
| W | Trp | tryptophane |
| Y | Tyr | tyrosine |

L'héliomicine est un peptide amphiphile présentant une structure tridimensionnelle du type CSαβ. La séquence en acides aminés de l'héliomicine représentée dans la liste de séquence sous le numéro SEQ ID NO :1 est la suivante :

La Demanderesse a maintenant isolé, à partir de l'hémolymphe de larves immunisées du lépidoptère *Archeoprepona demophoon,* un homologue de l'héliomicine. Ce peptide, désigné Ard1, a été caractérisé par séquençage et mesure de masse. La séquence en acides aminés de Ard1 est représentée dans la liste de séquence sous le numéro SEQ ID NO :2

La séquence de Ard1 diffère de celle de l'héliomicine en 2 positions : un acide aspartique (Asp) en position 17 dans l'héliomicine est remplacé par une asparagine (Asn) et une glycine (Gly) en position 20 est remplacée par une alanine (Ala). Les codons correspondants ont été modifiés dans le vecteur d'expression de l'héliomicine pSEA2 et le peptide Ard1 a été produit et sécrété par la levure *S.cerevisiae*.

pSEA2 est un vecteur d'expression de levure porteur du promoteur *MF*α1 et des séquences pré de *BGL*2 et pro de *MF*α1 permettant la sécrétion du peptide dans le milieu de culture (Lamberty et al., 1999, J. Biol. Chem., 274, 9320-9326).

Après purification par HPLC, l'activité antifongique (activité anti-*Candida albicans* et anti-*Aspergillus fumigatus)* de Ard1 a été comparée à celle de l'héliomicine. L'activité anti-*Candida albicans* de Ard1 est de 4 à 8 fois supérieure à celle de l'héliomicine. L'activité anti-*Aspergillus fumigatus* de Ard1 est 2 fois supérieure à celle de l'héliomicine.

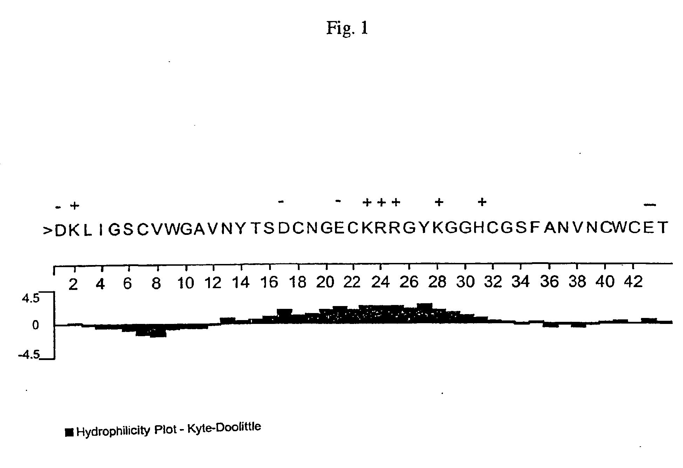

La Demanderesse a analysé la charge et l'hydrophobicité de l'héliomicine et du peptide Ard1. Le profil d'hydrophobicité représenté à la figure 1 en annexe a été réalisé par la méthode de Kyte et Doolittle (1982, J. Mol. Biol.,157, 105-132).

L'héliomicine et son homologue Ard1 possèdent deux régions à caractère plutôt hydrophobe séparées par une région à caractère plutôt hydrophile. Les régions N et C terminales présentent un caractère plutôt hydrophile. En outre la région centrale à caractère hydrophile présente une charge nette positive. La figure 1 indique la charge des acides aminés de la séquence de l'héliomicine.

Le remplacement de l'acide aspartique dé l'héliomicine en asparagine (position 17) dans l'homologue naturel Ard1 augmente le caractère cationique du peptide (+1 par rapport à l'héliomicine). D'autres mutations visant à augmenter la charge positive et l'hydrophobicité ont été réalisées dans l'héliomicine et son homologue Ard1 par mutagenèses dirigées générées par PCR ou par clonage de fragments synthétiques.

Les travaux de recherche réalisés dans le cadre de la présente invention ont donc consisté à effectuer des mutations notamment dans les régions hydrophobes et chargés de façon à augmenter la charge et/ou l'hydrophobicité des peptides sans modifier ou en améliorant leur caractère amphiphile, et ainsi disposer de peptides présentant des propriétés antifongiques et/ou antibiotiques améliorées par rapport à l'héliomicine.

Ce but est atteint grâce à un peptide dérivé de l'héliomicine de formule SEQ ID No. 1 : par substitution de un à plusieurs acides aminés. Les peptides de l'invention répondent à la formule (I), où « X » représente un acide aminé :

X₁ X₂ X₃ X₄ X₅ X₆ C₇ X₈ X₉ X₁₀ X₁₁ X₁₂ X₁₃ X₁₄ X₁₅ X₁₆ X₁₇ C₁₈ X₁₉ X₂₀X₂₁ C₂₂ X₂₃ X₂₄ X₂₅ X₂₆ X₂₇ X₂₈ X₂₉ X₃₀ X₃₁ C₃₂ X₃₃ X₃₄ X₃₅ X₃₆ X₃₇ X₃₈ X₃₉ C₄₀ X₄₁ C₄₂ X₄₃ X₄₄ (I)

dans laquelle :
- X₁, X₁₇, X₂₁, X₄₃ sont des acides aminés acides,
- X₁₆, X₄₄ sont des acides aminés polaires petits,
- X₁₉ est un acide aminé polaire large,
- X₃₆ est un acide aminé petit ou faiblement hydrophobe,
- X₃₈ est un acide aminé faiblement hydrophobe ou petit,
lesdites substitutions étant telles que :
- l'un au moins des X₁, X₁₇, X₂₁, X₄₃ est un acide aminé basique ou polaire avantageusement polaire large, et/ou
- l'un au moins des acides aminés X₁₆, X₄₄ est un acide aminé basique ou un acide aminé polaire large, et/ou
- X₁₉ est un acide aminé basique, et/ou
- l'un au moins des acides aminés X₃₆, X₃₈ est un acide aminé fortement hydrophobe,
et dans laquelle, les autres acides aminés (X) ont les significations suivantes :
- X₁₃, X₃₇, X₃₉ représentent des acides aminés polaires larges,
- X₆, X₁₅, X₃₄ représentent des acides aminés polaires petits,
- X₂, X₂₃, X₂₄, X₂₅, X₂₈, X₃₁ représentent des acides aminés basiques,
- X₃, X₄, X₈, X₁₂ représentent des acides aminés hydrophobes,
- X₉, X₁₄, X₂₇, X₃₅, X₄₁ représentent des acides aminés hydrophobes aromatiques,
- X₅, X₁₀, X₁₁, X₂₀, X₂₆, X₂₉, X₃₀, X₃₃ représentent des acides aminés petits,
- C₇, C₁₈, C₂₂, C₃₂, C₄₀, C₄₂ représentent des cystéines.

Ainsi, dans les peptides de l'invention de formule (I), lorsque :
- tout ou partie des X₁, X₁₇, X₂₁, X₄₃ n'est pas un acide aminé basique ou polaire avantageusement polaire large, il(s) est (sont) un (des) acide(s) aminé(s) acide(s),
- tout ou partie des X₁₆, X₄₄ n'est pas un acide aminé basique ou polaire large, il(s) est (sont) un (des) acide(s) aminé(s) polaire(s) petit(s),
- X₁₉ n'est pas un acide aminé basique, il est un acide aminé polaire large,
- X₃₆ n'est pas un acide aminé fortement hydrophobe, il est un acide aminé petit ou faiblement hydrophobe,
- X₃₈ n'est pas un acide aminé fortement hydrophobe, il est un acide faiblement hydrophobe ou petit.

Les peptides de l'invention possèdent la structure CSαβ de l'héliomicine, car les substitutions ne concernent pas les cystéines C₇, C₁₈, C₂₂, C₃₂, C₄₀, C₄₂.

Un premier groupe préféré de peptides selon l'invention est celui dans lequel l'un au moins des X₁, X₁₇, X₄₃ est un acide aminé basique ou polaire avantageusement polaire large, et X₂₁ est un acide aminé acide capable d'établir des liaisons ioniques avec l'un au moins des X₂₃, X₂₄ et X₂₅ qui sont des acides aminés basiques. En effet, ces liaisons sont susceptibles de participer à la stabilisation de la structure CSαβ des peptides de l'invention.

Un deuxième groupe préféré de peptides selon l'invention est celui dans lequel l'un au moins des X₃₆ et X₃₈ est un acide aminé fortement hydrophobe non aromatique.

Un troisième groupe préféré de peptides selon l'invention est celui dans lequel X₁₇ est l'asparagine ou l'arginine, X₄₃ est l'acide glutamique et dans lequel :
- X₃₆ est la leucine ou l'isoleucine, et/ou
- X₁₉ est l'arginine, et/ou
- X₁₆ est l'arginine.

Un quatrième groupe préféré de peptides selon l'invention est celui dans lequel X₁₇ est l'acide aspartique, X₄₃ est l'acide glutamique et dans lequel :
- X₃₆ est la leucine ou l'isoleucine, et/ou
- X₁₉ est l'arginine, et/ou
- X₁₆ est l'arginine.

Un cinquième groupe préféré de peptides selon l'invention est celui dans lequel X₄₃ est la glutamine, X₁₇ est l'asparagine et dans lequel :
- X₃₆ est la leucine ou l'isoleucine, et/ou
- X₁₉ est l'arginine.

Un sixième groupe préféré de peptides selon l'invention est celui dans lequel X₄₃ est la glutamine et X₁₇ est l'acide aspartique.

Un septième groupe préféré de peptides selon l'invention est celui dans lequel X₄₃ est la glutamine, X₁₇ est l'acide aspartique et dans lequel :
- X₁ est l'asparagine, et/ou
- X₃₆ est la leucine ou l'isoleucine.

On entend plus particulièrement par :
- acides aminés basiques : l'arginine, la lysine ou l'histidine,
- acides aminés hydrophobes :
   . non aromatiques : la méthionine, la valine, la leucine, l'isoleucine, étant entendu que la leucine et l'isoleucine sont des acides aminés fortement hydrophobe et que la méthionine et la valine sont des acides aminés faiblement hydrophobes,
   . aromatiques : la phénylalanine, la tyrosine, ou le tryptophane, qui sont des acides aminés fortement hydrophobes,
- acides aminés acides : l'acide aspartique ou l'acide glutamique,
- acides aminés polaires larges : la glutamine ou l'asparagine,
- acides aminés polaires petits : la sérine ou la thréonine,
- acides aminés polaires : acides aminés polaires petits et larges,
- acides aminés petits : la glycine ou l'alanine.

Les peptides de l'invention peuvent être préparés par synthèse chimique ou par génie génétique par des techniques bien connues de l'homme du métier.

Trois types de mutations ont plus particulièrement été générées :
- des acides aminés acides ont été remplacés par des acides aminés polaires, comme les mutations Asp1 en Asn, Asp17 en Asn, Glu43 en Gln, et
- des acides aminés polaires de préférence polaires larges ont été remplacés par des acides aminés basiques, comme les mutations Asn13 en Arg, Ser16 en Arg, Asn 17 en Arg (Ard1), Asn 19 en Arg, Thr 44 en Arg.
- des mutations visant à augmenter l'hydrophobicité ont également été générées, comme les mutations Gly10 en Leu, Ala36 en Leu ou Ile et Val38 en Ile.

Des peptides préférés dérivés de l'héliomicine selon l'invention présentent les séquences en acide aminés suivantes :

Des peptides préférés dérivés de Ard1 selon l'invention présentent les séquences en acides aminés suivantes :

L'invention concerne aussi des équivalents fonctionnels des peptides ci-dessus. Il s'agit par exemple de fragments des peptides ci-dessus ou de modifications résultant des processus post-traductionnels comme des glycosylation ou des modifications chimiques telles que l'amidation, l'acétylation, l'acylation, le couplage avec des lipides ou des sucres, le couplage avec des nucléotides, etc.

Les équivalents fonctionnels comprennent également des peptides de l'invention dont un ou plusieurs des acides aminés des acides aminés sont des énantiomères, des diastéréoisomères, des acides aminés naturels de conformation D, des acides aminés rares notamment l'hydroxyproline, la méthyllysine, la diméthyllysine et les acides aminés synthétiques, notamment l'ornithine, la norleucine, la cyclohexylalanine et les oméga-aminoacides. L'invention couvre également les rétropeptides et les rétro-inversopeptides.

Les.peptide de formule (I) peuvent comprendre également à l'une ou l'autre de leurs extrémités N- ou C-terminale un ou plusieurs acides aminés qui n'interfèrent pas avec la structure de la formule (I). En effet, l'invention vise bien entendu des peptides présentant une structure tridimensionnelle du type comportant une hélice α et un feuillet β antiparallèle reliés par trois ponts disulfures, comme l'héliomicine.

Le tableau 1 ci-dessous rapporte des mutations effectuées sur les acides aminés en positions 1, 6, 13, 16, 19, 36, 38, 43 et 44 de l'héliomicine, ainsi que l'activité antifongique sur *C.albicans* (C.a) et *A.fumigatus* (A.f) des peptides obtenus.

**Tableau 1**

| position | 1 | 6 | 13 | 16 | 19 | 36 | 38 | 43 | 44 | | |
|---|---|---|---|---|---|---|---|---|---|---|---|
| Héliomicine | D | S | N | S | N | A | V | E | T | Activité⁺ | |
| Mutants | | | | | | | | | | C.a | A.f |
| pEM37 | N | | | | | | | | | 2 | - |
| pEM38 | | T | | | | | | | | 1 | 1 |
| pEM45 | | | R | | | | | | | 0,5 | <<<1 |
| pEM43 | | | | T | | | | | | 1 | 2 |
| pEM42 | | | | R | | | | | | 8 | 1 |
| pEM44 | | | | | R | | | | | 8 | 2 |
| pEM22 | | | | | | I | | | | 1-2 | 4-8 |
| pEM23 | | | | | | | I | | | 1 | 2 |
| pEM25 | | | | | | L | | | | 10 | 6 |
| pEM24 | | | | | | L | I | | | 4 | 8 |
| pEM7 | | | | | | | | | R | 4-8 | 1 |
| pEM21 | | | | | | | | Q | | 2 | 10-20 |
| pEM39 | N | | | | | | | Q | | 2 | 4 - 8 |
| pEM61 | N | | | | | | L | Q | | 5-10 | 3-6 |
| pEM62 | N | | | | | | I | Q | | 3-6 | 2-4 |

| | | | | | | | | | | | |
|---|---|---|---|---|---|---|---|---|---|---|---|
| + activité relative par rapport à l'héliomicine. | | | | | | | | | | | |

Le tableau 2 ci-dessous rapporte des mutations effectuées sur les acides aminés en positions 1, 10, 16, 17, 19, 36, 38, 43 et 44 du peptide Ard1, ainsi que l'activité antifongique sur *C.albicans* (C.a) et *A.fumigatus* (A.f) des peptides obtenus.

**Tableau 2**

| position | 1 | 10 | 16 | 17 | 19 | 36 | 38 | 43 | 44 | | |
|---|---|---|---|---|---|---|---|---|---|---|---|
| Ard1 | D | G | S | N | N | A | V | E | T | Activité⁺ | |
| Mutants | | | | | | | | | | C.a | A.f |
| pEM40 | N | | | | | | | | | 2 | 1 |
| pEM50 | | | R | | | | | | | 1-2 | 2 |
| pEM56 | | L | | | | | | | | 1-2 | 0,5 |
| pEM52 | | | | R | | | | | | 1-2 | 0,5 |
| pEM51 | | | | | R | | | | | 2-4 | 1 |
| pEM32 | | | | | | I | | | | 1 | 2 |
| pEM33 | | | | | | | I | | | 1 | 1 |
| pEM34 | | | | | | L | I | | | 4 | 4 |
| pEM35 | | | | | | L | | | | 4 | 2-4 |
| pEM31 | | | | | | | | Q | | 2 | 4-8 |
| pEM30 | | | | | | | | | R | 4 | 0,5-1 |
| pEM46 | | | | | | L | | Q | | 4-8 | 6-8 |
| pEM47 | | | | | | I | | Q | | 2-4 | 8 |
| pEM48 | | | | | | L | | | R | 6-12 | 1-2 |
| pPEM49 | | | | | | I | | | R | 3 | 1-2 |
| pEM54 | | L | | | | L | | | | 1 | 1 |
| pEM57 | | L | | | | | | Q | | 1 | 8 |
| pEM55 | | L | | | | L | | Q | | 1-2 | 2-7 |

| | | | | | | | | | | | |
|---|---|---|---|---|---|---|---|---|---|---|---|
| + activité relative par rapport à Ard1. | | | | | | | | | | | |

Les différents mutants ont été produits dans la levure *S.cerevisiae*, purifiés par HPLC et leur activité antifongique (*C.albicans* et *A.fumigatus*) a été comparée à celle de l'héliomicine ou du peptide Ard1.

Les tableaux 1 et 2 ci-dessus montrent un gain d'activité sur au moins un des deux champignons testés pour tous les mutants d'augmentation de charge positive à l'exception du mutant Asn 13 en Arg (pEM45). Les autres mutants sont tous localisés dans des régions hydrophiles. La majorité des mutants ont une activité accrue sur *C.albicans* (Ser16 en Arg, Asn17 en Arg (Ard1), Asn19 en Arg, Thr44 en Arg). Une seule mutation (Glu43 en Gln) permet un gain d'activité significativement important sur *A. fumigatus*.

En ce qui concerne les mutations d'augmentation d'hydrophobicité, la modification de l'Ala36 en Leu (pEM35) permet le meilleur gain d'activité sur *C.albicans* et *A.fumigatus.* Les mutations Gly10 en Leu et Va138 en Ile n'ont pas d'effet significatif sur l'activité antifongique de l'héliomicine et de Ard1.

Les mutants les plus actifs d'augmentation d'hydrophobicité ont été associés aux mutants d'augmentation de charge. Des effets cumulatifs ont ainsi été observés.

L'invention a aussi pour objet l'utilisation des peptides ci-dessus, pour prévenir ou traiter une infection fongique et/ou bactérienne tant chez l'homme et l'animal que chez les plantes. L'invention a donc pour objet une composition, plus particulièrement pharmaceutique antifongique et/ou antibactérienne, comprenant à titre d'agent actif au moins un peptide tel que défini précédemment, avantageusement associé dans ladite composition avec un véhicule acceptable.

Le véhicule est choisi en fonction du type d'application de la composition à titre pharmaceutique ou agronomique.

L'invention concerne tout particulièrement les applications pharmaceutiques chez l'homme et l'animal de ces peptides et des compositions les contenant, mais elle s'intéresse aussi aux applications agronomiques. En effet, les peptides de l'invention sont utiles pour rendre des plantes résistantes contre des maladies notamment fongiques et bactériennes. Une première forme de mise en oeuvre de cette application agronomique, consiste à appliquer sur les plantes une quantité efficace de peptides ou d'une composition les contenant. Une seconde forme de mise en oeuvre de cette application agronomique consiste à transformer des cellules végétales ou des plantes avec une séquence d'acide nucléique codant pour un peptide de l'invention de façon à conférer aux plantes une résistance aux maladies.

D'autres avantages et caractéristiques de l'invention apparaîtront des exemples qui suivent concernant la préparation du peptide Ard1 et d'analogues de l'héliomicine et de Ard1 et leur activité antifongique et dans lesquels il sera fait référence aux dessins en annexe dans lesquels :
- la figure 1 représente le profil d'hydrophobicité du peptide Héliomicine par la méthode de Kyte et Doolittle (1982, J. Mol. Biol., 157, 105-132);
- la figure 2 représente les activités (pourcentage de survie par rapport aux jours post-infection) des peptides Héliomicine et Ard 1 dans le modèle d'infection disséminé à *Candida albicans*;
- la figure 3 représente les activités (scores de morbidité par rapport aux jours post-infection) des peptides Héliomicine et Ard 1 dans le modèle d'infection disséminé à *Candida albicans;*
- la figure 4 représente les activités (pourcentage de survie par rapport aux jours post-infection) des peptides pEM24, pEM30, pEM31 et pEM35 dans le modèle d'infection disséminé à *Candida albicans*;
- la figure 5 représente les activités (scores de morbidité par rapport aux jours post-infection) des peptides pEM24, pEM30, pEM31 et pEM35 dans le modèle d'infection disséminé à *Candida albicans;*
- la figure 6 représente les activités (pourcentage de survie par rapport aux jours post-infection) des peptides pEM31, pEM35, pEM46 et pEM51 dans le modèle d'infection disséminé à *Candida albicans;*
- la figure 7 représente les activités (scores de morbidité par rapport aux jours post-infection) des peptides pEM31, pEM35, pEM46 et pEM51 dans le modèle d'infection disséminé à *Candida albicans;*
- la figure 8 représente les activités (pourcentage de survie par rapport aux jours post-infection) du peptide pEM35 dans le modèle d'infection disséminé à *Candida albicans*;
- la figure 9 représente les activités (pourcentage de survie par rapport aux jours post-infection) des peptides pEM35 et pEM51 dans le modèle d'infection disséminé à *Scedosporium inflatum;*
- la figure 10 représente les activités (indice de morbidité par rapport aux jours post-infection) des peptides pEM35 et pEM51 dans le modèle d'infection disséminé à *Scedosporium inflatum*;
- la figure 11 représente l'évolution pondérale en fonction du temps des souris saines traitées avec le peptide pEM51;
- la figure 12 représente l'évolution pondérale en fonction du temps des souris saines traitées avec les peptides pEM35 et pEM51;
- la figure 13 représente la cinétique de fongicidie des peptides pEM35 et pEM51 contre *Candida albicans* IHEM 8060;

### Exemple 1 : Isolement de Ard1 à partir de l'hémolymphe prélevée chez des larves immunisées du lépidoptère A. demophoon.

### 1) Induction de la synthèse biologique d'une substance antifongique dans l'hémolymphe d' A. demophoon.

Les larves matures de 4ème stade du lépidoptère *A. demophoon* ont été immunisées par deux injections de 20 µl d'une solution de PBS contenant des bactéries à Gram positif (*M. luteus et S. aureus)* et à Gram négatif (*P*. *aeruginosa),* des spores de champignon filamenteux (A. *fumigatus*) et des levures (*C. albicans*). Les bactéries sont préparées à partir de cultures réalisées en milieu de Luria-Bertani durant 12 heures à 37°C. Les levures sont préparées à partir de cultures réalisées en milieu de Sabouraud durant 12 heures à 30°C. Les spores d'*A. fumigatus* sont prélevées d'un stock congelé à -80°C. Les animaux ainsi infectés ont été conservés pendant 24 heures sur leur plante hôte, dans un espace ventilé. Avant le prélèvement de l'hémolymphe, les larves ont été refroidies sur de la glace.

### 2) Préparation du plasma.

L'hémolymphe (environ 160 µl par larve, pour un total de 81 spécimens) a été collectée par excision d'un appendice abdominal et recueillie dans des tubes de microcentrifugation en polypropylène de 1,5 ml refroidis dans de la glace et contenant de l'aprotinine comme inhibiteur de protéases (20 µg/ml en concentration finale) et de la phénylthiourée comme inhibiteur de la mélanisation (concentration finale de 40 µM). L'hémolymphe (13 ml) ainsi collectée à partir des larves immunisées a été centrifugée à 8000 rpm pendant 1 min à 4°C afin d'éliminer les hémocytes. Le surnageant de centrifugation a été alors centrifugé à 12000 rpm. L'hémolymphe dépourvue des cellules sanguines a été conservée à -80°C jusqu'à son utilisation.

### 3) Acidification du plasma.

Après décongélation rapide, le plasma d'*A. demophoon* a été acidifié jusqu'à pH 3 avec une solution d'acide trifluoroacétique à 1% (volume à volume) contenant de l'aprotinine (20 µg/ml en concentration finale) et la phénylthiourée (concentration finale de 40 µM). L'extraction en condition acide du peptide a été réalisée pendant 30 min sous agitation légère dans un bain d'eau glacée. L'extrait obtenu a été ensuite centrifugé à 4°C pendant 30 min à 10000g.

### 4) Purification des peptides.

### a) Prépurification par extraction en phase solide.

Une quantité d'extrait équivalente à 5 ml d'hémolymphe a été déposée sur un support de 2 g de phase inverse, tel que commercialisé sous la forme de cartouche (Sep-Pak™ C₁₈, Waters Associates), équilibré avec de l'eau acidifiée (TFA 0,05 %). Les molécules hydrophiles ont été éliminées par un simple lavage avec de l'eau acidifiée. L'élution du peptide a été réalisée par une solution d'acétonitrile à 60% préparée dans le TFA 0,05%. La fraction éluée à 60% d'acétonitrile a été séchée sous vide dans le but d'éliminer l'acétonitrile et le TFA puis elle a été reconstituée dans de l'eau acidifiée (TFA 0,05%) stérile avant d'être soumise à la première étape de purification.

### b) Purification par chromatographie liquide à haute performance (HPLC) sur colonne de phase inverse.

- première étape : la fraction contenant le peptide a été analysée par chromatographie de phase inverse sur une colonne préparative Aquapore RP-300 C₈ (Brownlee™, 220 x 10 mm, 300 Å ), l'élution a été réalisée par un gradient d'acétonitrile dans le TFA 0,05% de 2% à 10% en 5 minutes, puis de 10 à 25% en 30 minutes, puis de 25% à 35% en 40 minutes, puis de 35% à 60% en 50 minutes, pour une durée totale de 125 minutes à un débit constant de 2,5 ml/min. Les fractions ont été collectées manuellement en suivant la variation de l'absorbance à 225 nm. Les fractions recueillies ont été asséchées sous vide, reconstituées avec de l'eau ultrapure et analysées pour leur activité antifongique en utilisant le test décrit ci-dessous.
- seconde étape : la fraction antifongique éluée à 27% d'acétonitrile correspondant au peptide a été analysée sur une colonne analytique de phase inverse Aquapore RP-300 C₈ (Brownlee™, 220 x 4,6 mm, 300 Å), en utilisant un gradient linéaire diphasique d'acétonitrile de 2% à 23% en 5 min et de 23 à 31% en 50 min dans le TFA 0,05% avec un débit constant de 0,8 ml/min. Les fractions ont été collectées manuellement en suivant la variation de l'absorbance à 225 nm. Les fractions recueillies ont été asséchées sous vide, reconstituées avec de l'eau ultrapure et analysées pour leur activité antifongique dans les conditions décrites ci-dessous.
- troisième étape : la fraction antifongique contenant le peptide a été purifiée jusqu'à homogénéité sur une colonne de phase inverse Narrowbore Delta-Pak™ HPI C₁₈ (Waters Associates, 150 x 2 mm) en utilisant un gradient linéaire diphasique d'acétonitrile de 2% à 22% en 5 min et de 22 à 30% en 50 min dans le TFA 0,05% avec un débit constant de 0,25 ml/min à une température contrôlée de 30°C. Les fractions ont été collectées manuellement en suivant la variation de l'absorbance à 225 nm. Les fractions recueillies ont été asséchées sous vide, reconstituées avec de l'eau ultrapure filtrée et analysées pour leur activité antifongique.

### Exemple 2 : Caractérisation structurale du peptide de ArdI.

### 1) Vérification de la pureté par Spectrométrie de masse Maldi-TOF (Matrix Assisted Laser Desorption Ionization-Time Of Flight).

Le contrôle de pureté a été effectué sur un spectromètre de masse MALDI-TOF Bruker Biflex (Bremen, Allemagne) en mode linéaire positif (voir section 3 ci-dessous).

### 2) Détermination du nombre des cystéines : réduction et S-pyridyléthylation.

Le nombre de résidus cystéine a été déterminé sur le peptide natif par réduction et S-pyridyléthylation. 400 pmoles de peptide natif ont été réduites dans 40 µl de tampon Tris/HCl 0,5 M, pH 7,5 contenant 2 mM d'EDTA et 6 M de chlorure de guanidinium en présence de 2 µl de dithiothréitol (2,2 M). Le milieu réactionnel a été placé sous atmosphère d'azote. Après 60 min d'incubation à l'obscurité, 2 µl de 4-vinylpyridine fraîchement distillée ont été ajoutés à la réaction qui a été alors incubée durant 10 min à 45°C à l'obscurité et sous atmosphère d'azote. Le peptide pyridyléthylé a été ensuite séparé des constituants du milieu réactionnel par chromatographie de phase inverse sur une colonne analytique de phase inverse Aquapore RP-300 C₈ (Brownlee™, 220 x 4,6 mm, 300 Å), en utilisant un gradient linéaire d'acétonitrile en présence de TFA 0,05% de 2 à 52% pendant 70 minutes.

### 3) Détermination de la masse du peptide natif, du peptide S-pyridyléthylé et des fragments de protéolyse par spectrométrie de masse MALDI-TOF (Matrix Assisted Laser Desorption Ionization-Time Of Flight).

Les mesures de masses ont été effectuées sur un spectromètre de masse MALDI-TOF Bruker Biflex (Bremen, Allemagne) en mode linéaire positif. Les spectres de masse ont été calibrés de façon externe avec un mélange standard de peptides de m/z connues, respectivement 2199,5 Da, 3046,4 Da et 4890,5 Da. Les différents produits à analyser ont été déposés sur une fine couche de cristaux d'acide α-cyano-4-hydroxycinnamique obtenue par une évaporation rapide d'une solution saturée dans l'acétone. Après séchage sous un léger vide, les échantillons ont été lavés par une goutte d'acide trifluoroacétique à 0,1% avant d'être introduits dans le spectromètre de masse.

### 4) Séquençage par dégradation d'Edman

Le séquençage automatique par dégradation d'Edman du peptide natif, du peptide S-pyridyléthylé et des différents fragments obtenus après les différents clivages protéolytiques et la détection des dérivés phénylthiohydantoines ont été réalisés sur un séquenceur ABI473A (PEApplied Biosystems division de Perkin Elmer).

### 5) Clivages protéolytiques.

Confirmation de la séquence peptidique dans la région C-terminale. 200 pmoles de peptide réduit et S-pyridyléthylé ont été incubées en présence de 5 pmoles d'endoprotéinase-Lys-C (*Acromobacter* protease I, clivage spécifique des résidus lysine du côté C-terminal, Takara, Otsu) selon les conditions préconisées par le fournisseur (Tris-Hcl 10 mM pH 9 en présence de Tween 20 à 0,01%). Après arrêt de la réaction avec du TFA 1%, les fragments peptidiques ont été séparés par HPLC en phase inverse sur une colonne de type Narrowbore Delta-Pak™ HPIC₁₈ (Waters Associates, 150 x 2 mm) dans un gradient linéaire d'acétonitrile de 2 à 60% en 80 min dans le TFA 0,05% avec un débit de 0,2 ml/min et une température constante de 37°C. Les fragments obtenus ont été analysés par spectrométrie de masse MALDI-TOF et le peptide correspondant au fragment C-terminal a été séquencé par dégradation d'Edman.

### Exemple 3 : Production du peptide Ard1 dans la levure S.cerevisiae.

### 1) Construction du vecteur pEM2 permettant l'expression et la sécrétion par la levure S. cerevisiae de l'analogue Ard1.

A partir du vecteur d'expression de l'héliomicine pSEA2 décrit par Lamberty et al. (1999, J. Biol. Chem., 274, 9320-9326), une mutagenèse dirigée a été réalisée par PCR pour modifier les codons Asp17 en Asn et Gly20 en Ala. Un fragment porteur du promoteur MFα1, des séquences pre BGL2 et pro MFα1 et de la séquence codante de l'héliomicine jusqu'au site *Sac*II a été amplifié par PCR avec les oligonucléotides EM 72 et EM 89. Les mutations des codons 17 et 20 ont été introduites dans l'oligonucléotide EM89.

Le fragment amplifié par PCR a été digéré par les enzymes de restriction *Sph*I et *Sac*II et cloné dans le plasmide pSEA2 digéré par les mêmes enzymes et traité à la phosphatase alcaline. Le plasmide résultant pEM2 a été contrôlé par analyse de restriction et séquençage.

### 2) Transformation d'une souche de levure S.cerevisiae par le plasmide pEM2.

La souche de levure TGY48.1 (*MAT*α, ura3-Δ5, ,his, pra1, prb1, prc1, cps1, Reichhart et al., 1992, Invert. Reprod. Dev. **21**, 15-24) a été transformée par le plasmide pEM2. Les transformants ont été sélectionnés sur milieu sélectif YNBG supplémenté de 0,5% de casamino acides.

### Exemple 4 : Préparation des analogues de l'héliomicine pEM22, pEM24, pEM30, pEM31, pEM34, pEM35, pEM37, pEM46 et pEM48.

### 1) Construction des vecteurs pEM22 et pEM24.

Un fragment synthétique constitué des oligonucléotides EM25 et EM26 préalablement hybridés (chauffage à 100°C et diminution lente de la température jusqu'à 25°C) a été cloné dans le vecteur pSEA2 digéré par *Bam*HI et *Sal*I (remplacement de l'extrémité 3' de la séquence codant pour l'héliomicine, codon Ser34 jusqu'au codon stop). Ce fragment synthétique *Bam*HI*-Sal*I contient les sites de restriction *Xho*I et *Nhe*1*.* Le vecteur résultant pEGO1 a été contrôlé par analyse de restriction et séquençage.

Un fragment synthétique *Bam*HI-*Sal*I constitué des oligonucléotides EM119 et EM120 préalablement hybridés a été cloné dans le vecteur pEGO1. La réaction de ligation a été digérée par *Xho*I afin d'éliminer les plasmides n'ayant pas inséré le fragment synthétique EM119/EM120. Le plasmide résultant pEM22 a été contrôlé par analyse de restriction et séquençage. Une stratégie de clonage identique a été utilisée pour la construction de pEM24 en utilisant le couple d'oligonucléotides EM127 et EM128.

### 2) Construction des vecteurs pEM30, pEM31, pEM34, pEM35, pEM46 et pEM48.

Un fragment synthétique constitué des oligonucléotides EM25 et EM26 préalablement hybridés (chauffage à 100°C et diminution lente de la température jusqu'à 25°C) a été cloné dans le vecteur pEM2 digéré par *Bam*HI et *Sal*I (remplacement de l'extrémité 3' de la séquence codant pour Ard1, codon Ser34 jusqu'au codon stop). Ce fragment synthétique *Bam*HI-*Sal*I contient les sites de restriction *Xho*I et *Nhe*1. Le vecteur résultant pEM16 a été contrôlé par analyse de restriction et séquençage.

Un fragment synthétique *Bam*HI-*Sal*I constitué des oligonucléotides EM135 et EM136 préalablement hybridés a été cloné dans le vecteur pEM16. La réaction de ligation a été digérée par *Xho*I afin d'éliminer les plasmides n'ayant pas inséré le fragment synthétique EM135/EM136. Le plasmide résultant pEM30 a été contrôlé par analyse de restriction et séquençage. Une stratégie de clonage identique a été utilisée pour les constructions de pEM31 (EM117 /EM118), pEM34 (EM127/EM128), pEM35 (EM129/EM130), pEM46 (EM158/EM159), pEM48(EM162/EM163).

### 3) Construction du vecteur d'expression pEM37.

A partir du vecteur d'expression de l'héliomicine pSEA2, une mutagenèse dirigée a été réalisée par PCR pour modifier le codon Asp1 en Asn. Un fragment porteur de la fin de la séquence pro de MFα1 et de la séquence codante de l'héliomicine a été amplifié par PCR avec les oligonucléotides EM 137 et EM53. La mutation du codon Asp1 en Asn a été introduite dans l'oligonucléotide EM137.

Le fragment amplifié par PCR a été digéré par les enzymes de restriction *Hin*dIII et *Sal*I et cloné simultanément avec un fragment *Sph*I-*Hin*dIII de 1,2 kb porteur du promoteur MFα1, de la séquence pre de BGL2 et de la séquence pro de MFα1 jusqu'au site *Hin*dIII dans le vecteur pTG4812 (Michaud et al., 1996, FEBS Lett., 395, 6-10) digéré par *Sph*I et *Sal*I, et traité à la phosphatase alcaline. Le plasmide résultant pEM37 a été contrôlé par analyse de restriction et séquençage.

### Exemple 5: Tests de criblage réalisés sur les analogues de l'héliomicine.

### 1) Cultures.

Les clones de levure transformés par les plasmides d'expression de l'héliomicine et de ses analogues ont été cultivés en milieu sélectif (50 ml de YNBG + 0,5% de casamino acide) pendant 72 heures sous agitation à 29°C. Après centrifugation à 4000g pendant 30 min à 4°C, les surnageants ont été acidifiés à pH 3 avec de l'acide acétique.

Les surnageants ont ensuite été déposés sur un support de 360 mg de phase inverse Sep-Pak™ C₁₈ (Waters Associates), équilibré avec de l'eau acidifiée (TFA 0,05 %). Les molécules hydrophiles ont été éliminées par un simple lavage avec de l'eau acidifiée. Les peptides ont été élués par une solution d'acétonitrile à 60% préparée dans le TFA 0,05%. La fraction éluée à 60% d'acétonitrile a été séchée sous vide dans le but d'éliminer l'acétonitrile et le TFA puis elle a été reconstituée dans 1 ml de l'eau TFA 0,05% avant d'être soumise à la purification.

### 2) Purification par chromatographie liquide à haute performance (HPLC) sur colonne de phase inverse.

Selon le niveau de production obtenu pour chaque analogue, l'équivalent de 5 à 20 ml de surnageant prépurifié a été analysé par chromatographie de phase inverse sur une colonne semi-préparative Aquapore RP-300 C₈ (Brownlee™, 220 x 7 mm, 300 Å ), l'élution a été réalisée par un gradient d'acétonitrile dans le TFA 0,05% de 2% à 22% en 5 minutes, puis de 22 à 40% en 30 minutes après un isocratique de 2 minutes en 22%, à un débit constant de 1,4 ml/min. Les fractions éluées entre 27% et 38% d'acétonitrile ont été collectées manuellement en suivant la variation de l'absorbance à 225 nm.

### 3) Contrôle de masse de l'analogue.

1 µl des fractions majoritaires est dilué 2 fois dans de l'eau acidifiée par du TFA 0,05% et analysé en spectrométrie de masse Maldi-TOF. La fraction dont la masse mesurée correspond à la masse théorique a été asséchée sous vide, et reconstituée par ajout d'un volume d'eau ultrapure calculé comme décrit au paragraphe suivant.

### 4) Quantification de l'analogue en vue des tests d'activité.

Une courbe de calibration de la colonne semi-préparative Aquapore RP-300 C₈ est réalisée par injection de 5, 10, 20 et 25 µg d'héliomicine. L'intégration, le calcul des aires et de la pente de la droite est effectué par le logiciel Millenium (Waters). Par la suite, la quantification des analogues (en µg) est calculée par intégration automatique du pic du chromatogramme correspondant à l'analogue, par ce logiciel Le volume de reprise de l'échantillon après évaporation est calculé en fonction de la quantification ainsi obtenue de manière à ajuster la concentration en peptide à 1 µg/µl.

### 5) Tests d'activité anti-Candida albicans et anti-Aspergillus fumigatus.

Les activités anti-*Candida albicans* et anti-*Aspergillus fumigatus* des différents analogues sont évaluées par un test d'inhibition de croissance en milieu liquide réalisé en microplaques de 96 puits. L'activité des peptides purifiés est testée pour différentes dilutions de chaque peptide et elle est comparée à celles de l'héliomicine et de Ard I quantifiée dans les mêmes conditions.

### - Test anti-Candida albicans.

Le test d'activité est réalisé directement sur des levures issues d'un stock congelé à -80°C, dans du milieu Sabouraud contenant 15% de glycérol. La densité des levures dans le stock est ajustée à une densité optique de 0,4 DO à 600 nm. Après décongélation lente à température ambiante, la suspension de levures est ramenée par dilution à une densité optique de 1 mDO à 600nm dans du milieu Sabouraud et 90 µl de cette dilution sont déposés dans des puits de plaques de microtitration en présence de 10 µl d'échantillon à tester. Des cultures témoins sont réalisées systématiquement dans lesquelles 10 µl d'échantillons sont remplacés par 10 µl d'eau stérile. La stérilité des milieux est contrôlée en incubant 10 µl d'eau stérile en présence de 90 µl de milieu. Les échantillons sont incubés à 30°C pendant 40 h sous agitation faible et l'activité antifongique est quantifiée par la mesure de la densité optique à 600 nm.

### - Test anti-Aspergillus fumigatus.

Les spores d'*Aspergillus fumigatus* proviennent d'un stock de congélation à -80°C, titré à 10⁷ spores/ml dans une solution de glycérol à 25%. Après décongélation lente à température ambiante, les spores sont mises en suspension dans un milieu de culture PDB (12 g de milieu Potato Dextrose Broth, Difco, pour 1 1 d'eau déminéralisée. On dépose 10 µl de chaque échantillon dans des puits de plaques de microtitration en présence de 90 µl de milieu de culture PDB, supplémenté avec de la tetracycline (100 µg/ml) et du cefotaxime (1 µg/ml), et contenant les spores (à une concentration finale de 1000 spores/puit). Des cultures témoins sont réalisées systématiquement dans lesquelles 10 µl d'échantillons sont remplacés par 10 µl d'eau stérile. La stérilité des milieux est contrôlée en incubant 10 µl d'eau stérile en présence de 90 µl de milieu. Les échantillons sont incubés à 37°C pendant 24 h à 48 h en atmosphère humide et l'activité antifongique est quantifiée par un score de 0 à 9 prenant en compte la germination, la taille et la morphologie des hyphes sont déterminées sous la loupe binoculaire. La CMI correspond à un score de 4.

### 6) Contrôle de quantification.

Les solutions de peptides utilisées pour les tests d'activité sont systématiquement soumises à un contrôle de quantification par injection de 10 µl en HPLC, sur une colonne Narrowbore Delta-pack™ HPI C₁₈ préalablement étalonnée avec 2, 5, 7,5 et 10 µg d'héliomicine. La quantité de peptides effectivement déposée dans les puits est réajustée si besoin, lors de l'interprétation des résultats.

### Exemple 6 Efficacité in vivo.

### 1) Méthode - Modèle d'infection disséminée à Candida albicans.

L'héliomicine et ses analogues ont été testés pour leur activité antifongique *in vivo* dans un modèle d'infection disséminée à *Candida albicans*, léthale chez la souris. L'agent pathogène *Candida albicans* (souche IHEM 8060) est inoculé par voie intraveineuse (i.v.) à une dose de 2,5 x 10⁶ CFU/souris. Les peptides sont administrés par voie i.v. en 4 injections 6 h, 24 h, 48 h et 72 h après l'infection Les critères d'évaluation de l'activité utilisés sont l'étude de la survie et de la morbidité à 7 jours. Les scores de morbidité, qui prennent en compte l'état de santé générale (état du poil, mobilité, hydratation...) sont établis pour chaque souris par des valeurs comprises entre 0 à 5 et définies ci-après : 0 = morte, 1 = moribonde, 2 = très malade, 3 = malade, 4 = légèrement malade, 5 = saine. La somme des scores individuels est calculée pour chaque groupe, un score de 50 pour un groupe de 10 souris signifiant que toutes les souris sont saines.

### 2) Comparaison des activités de Ard 1 et de l'Héliomicine dans le modèle de candidose disséminée.

Dans un protocole type, des groupes de 10 souris mâles Swiss OF1 de 12 g sont infectées par voie i.v. par une dose 2,5 x 10⁶ CFU/souris. L'Héliomicine et Ard1 sont administrées par voie i.v. en 4 injections, 6h, 24h, 48h et 72 h après l'infection. Pour chaque peptide, 2 doses sont testées : 10 mg/kg et 30 mg/kg. Un groupe placebo est injecté avec le solvant des peptides du chlorure de sodium à 0,9%.

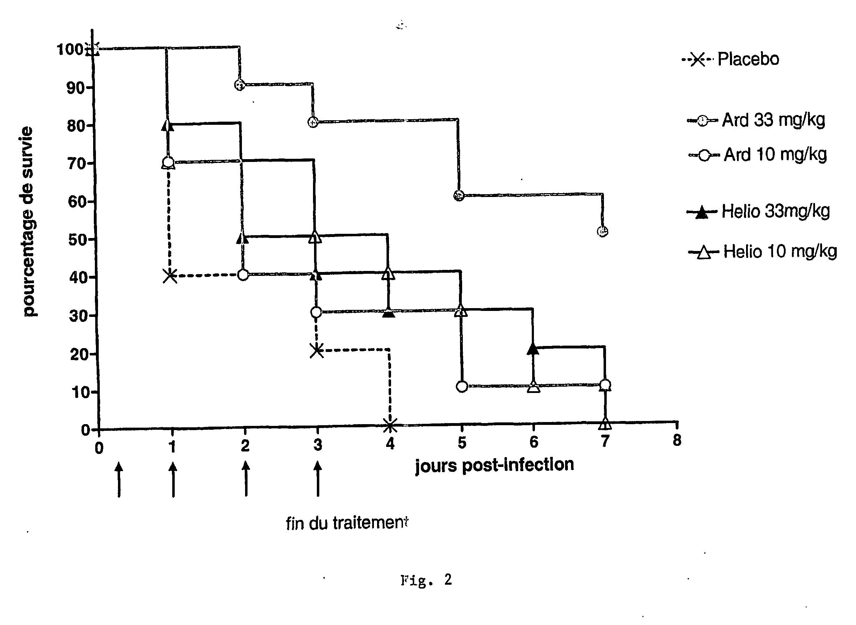

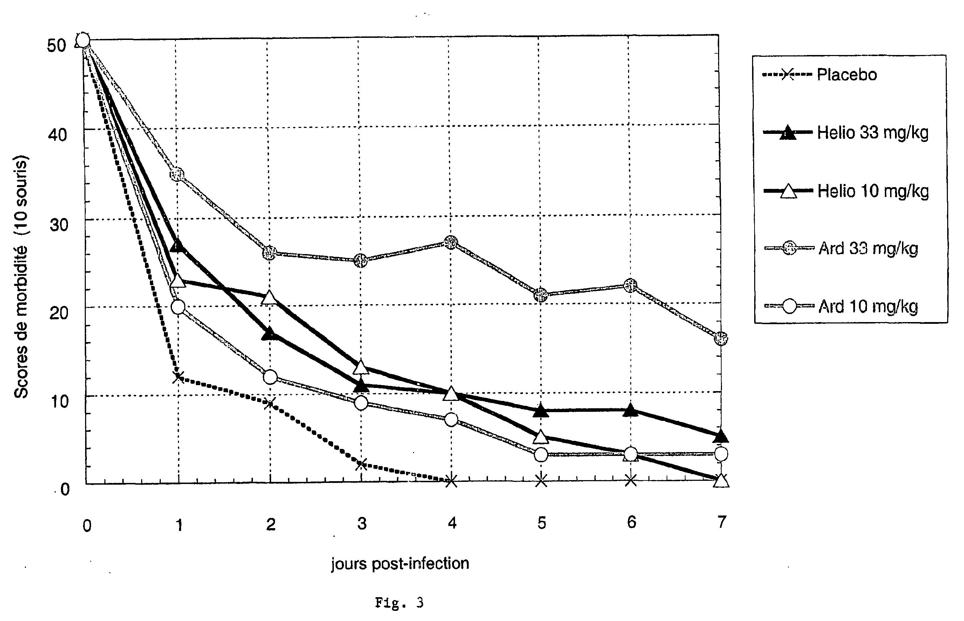

Les figures 2 et 3 en annexe représentent respectivement le pourcentage de survie et les scores de morbidité (10 souris) par rapport aux jours post-infection.

Dans ce modèle d'infection très sévère, léthale à 100% au jour 4 post-infection, 60% des souris du groupe placebo sont mortes dès le premier jour suivant l'infection.

On observe que l'Héliomicine, administrée à 10 et 30 mg/kg n'a pas d'effet significatif sur l'évolution de la survie, bien que les courbes soient toujours au dessus de la courbe relative au groupe placebo. La mortalité médiane intervient, respectivement pour les groupes traités avec des doses de 10 et 30 mg/kg d'Héliomicine, à 48 h et 60 h après l'infection , et les scores de morbidité sont de 0/50 et 5/50 au jour 7.

Dans ces conditions, le peptide Ard1 administré à la dose de 30 mg/kg retarde de 24 h l'apparition du premier décès. 5 souris sur 10 sont encore vivantes au jour 7, avec un score de morbidité de 16/50. La comparaison des courbes de survie (Meier-Kaplan) par le test statistique du logrank permet de conclure à une différence significative entre le groupe placebo et le groupe traité avec Ard1 à 30 mg/kg (p<0,001).

Ard1 administré à la dose de 10 mg/kg ne permet pas d'améliorer la survie et l'état général des souris, 50 % des souris étant mortes 2 jours après l'infection.

### 3) Comparaison des activités de Ard1 et des analogues pEM24, pEM30, pEM31 et pEM35 dans le modèle de candidose disséminée.

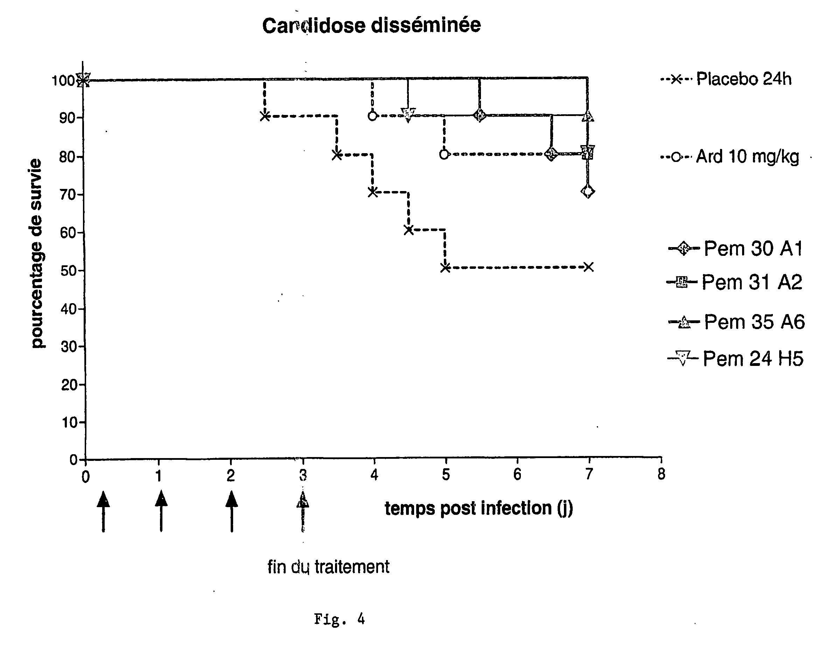

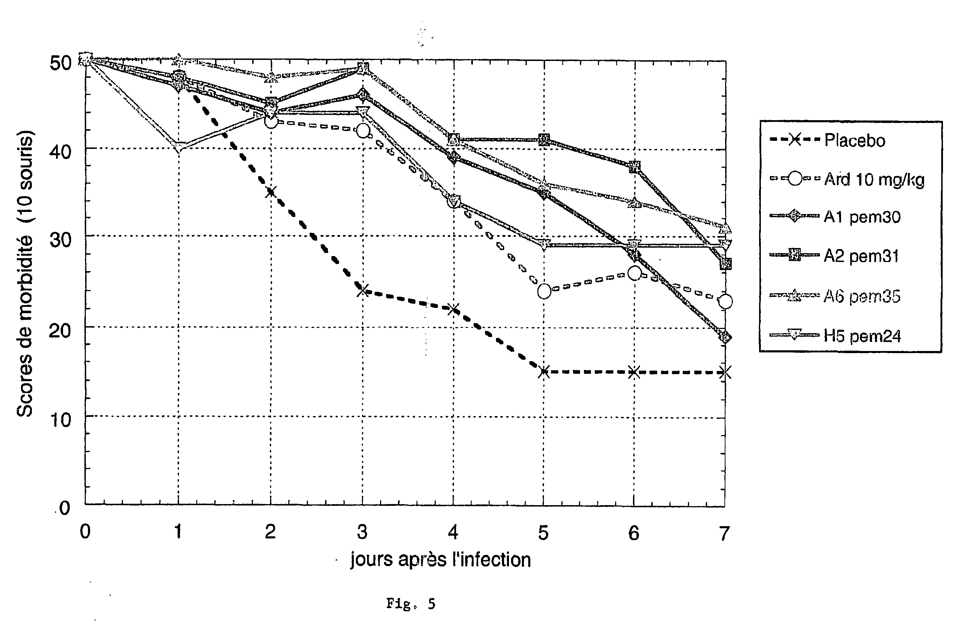

Les figures 4 et 5 en annexe représentent respectivement le pourcentage de survie et les scores de morbidité (10 souris) par rapport aux jours post-infection.

Dans cette expérience, l'inoculation des souris avec 2,5.10⁶ CFU/souris est léthale à 50 % au jour 5, dans le cas du groupe ayant reçu le traitement placebo. Les premiers décès ont lieu au jour 2,5 post-infection et la mortalité médiane se situe au jour 5 post infection. Au jour 7, 5 souris sont vivantes et le score de morbidité de 15/50.

Ard1 à la dose de 10 mg/kg retarde de 1,5 jours l'apparition du premier décès. 8 souris sont encore vivantes au jours 7 post-innoculation. La courbe de survie n'est cependant pas statistiquement différente de celle du groupe placebo (p= 0,2516).

Les quatre peptides testés à la dose de 10 mg/kg, pEM24 (H5), pEM30 (A1), pEM31 (A2) et pEM35 (A6), sont plus actifs que Ard1 : respectivement les délais d'apparition du 1^{er} décès et le nombre de souris vivantes au jour 7, sont de 3 jours et 7 souris pour le groupe traité avec le pEM24 (H5), 4 jours et 7 souris pour le groupe traité avec le pEM30 (A1), 5,5 jours et 8 souris pour le groupe traité avec le pEM31 (A2) et de 7 jours et 9 souris pour le groupe traité avec le pEM35 (A6). Chacun de ces peptide permet de maintenir les souris en bon état général pendant les 3 premiers jours, les scores de morbidité étant compris entre 42 et 48/50 au jour 3, comparé à 22/50 pour le groupe ayant reçu le placebo. L'état des souris décline 24 h après la 4^{ème} injection. Seul le groupe traité avec le pEM31 conserve un score de morbidité supérieur à 40/50 pendant 5 jours.

La comparaison statistique des courbes de survie avec celle du groupe placebo fait apparaître une différence significative pour le groupe traité avec le pEM35 avec un p égal à 0,041.

La comparaison statistique des courbes de survie avec celle du groupe placebo fait apparaître une différence significative pour les groupe traité avec le pEM31 au jour 8 avec un p égal à 0,0195.

Les activités relatives des peptides sont les suivantes : pEM31≥pEM35>pEM30>pEM24≥Ard1

### 4) Comparaison des activités de Ard1 et des analogues pEM31, pEM35, pEM37, pEM46 et pEM51 administrés à 5 mg/ml dans le modèle de candidose disséminée.

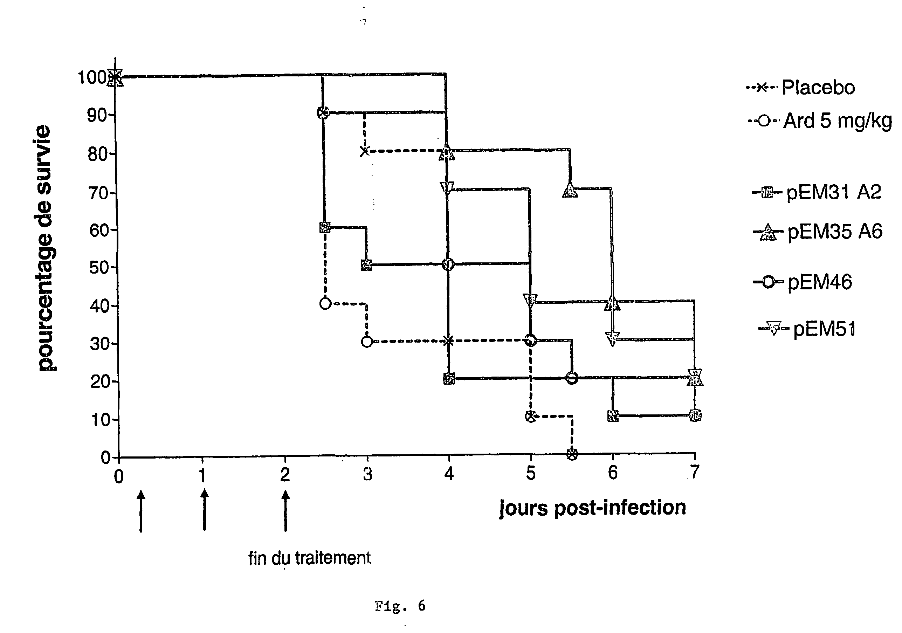

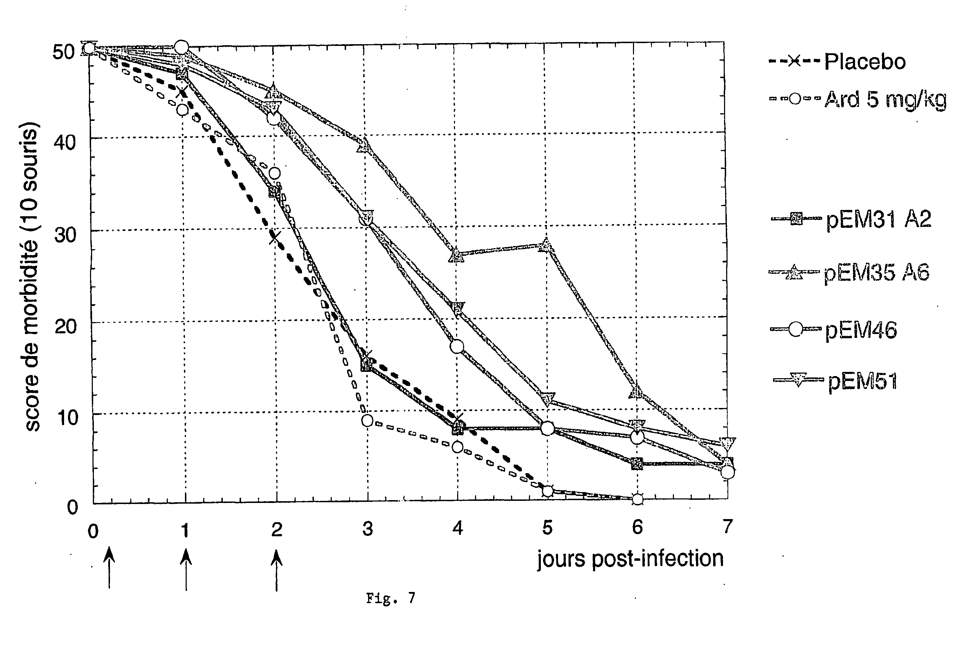

Les figures 6 et 7 en annexe représentent respectivement le pourcentage de survie et les scores de morbidité (10 souris) par rapport aux jours post-infection.

Dans cette expérience, l'inoculation de souris Swiss OF1 de 15 g avec 3.10⁶ CFU de *Candida albicans* induit, pour le groupe traité par le placebo, une mortalité de 50% au jour 4 après l'infection. Les 1^{er} décès interviennent 2,5 jours après l'infection, et 100% des souris sont mortes au jour 5,5.

Le traitement par Ard1 ainsi que par le pEM31 et le pEM46, administré en trois injections i.v. à la dose de 5 mg/kg n'augmente pas de façon significative la survie des souris par rapport au traitement placebo. Toutefois, le traitement par le pEM46 retarde la dégradation de l'état de santé des souris avec un indice de morbidité de 31/50, 3 jours après l'infection, comparé à 9/50 pour les souris du groupe placebo.

A cette dose, les traitements par le pEM51 et le pEM35 retardent de 1,5 jours l'apparition du 1^{er} décès ; la mortalité médiane intervient à 5 et 6 jours post-infection, respectivement, pour les groupes traités avec le pEM51 et le pEM35. Statistiquement, l'analyse des courbes de survie au jour 7, montre une différence significative par rapport au groupe placebo, avec un p égal à 0,015 pour le groupe traité avec le pEM51 et un p égal à 0,0004 pour le groupe traité avec le pEM35. L'état de santé général des souris traitées par le pEM35 est meilleur que celui des souris ayant reçu les autres traitements, avec un score de morbidité qui se maintient à 28/50 jusqu'au jour 5 après l'infection comparé à un score de 11/50 pour les souris ayant reçu le pEM51 et un score de 1/50 pour les souris ayant reçu le placebo.

Globalement, dans ce modèle de candidose disséminée, l'activité antifongique du pEM35 est supérieure à celle du pEM51, elle-même supérieure à l'activité du pEM46. A la dose de 5 mg/kg utilisée, les molécules Ard1 et le pEM31 ne sont pas efficaces.

### 5) Activité de l'analogue pEM35 dans le modèle de candidose disséminée.

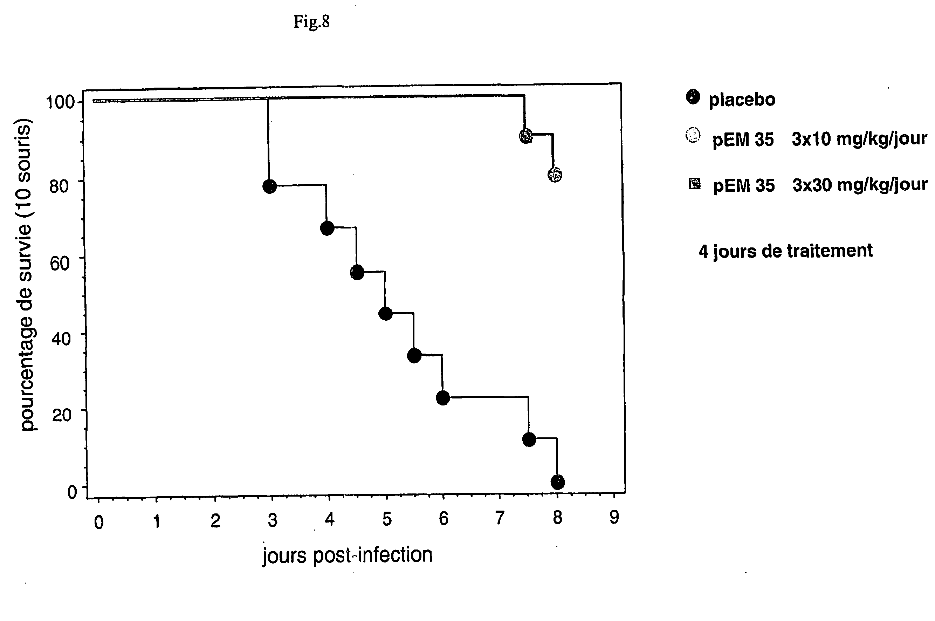

La figure 8 en annexe représente le pourcentage de survie (10 souris) par rapport aux jours post-infection. Dans cette expérience, l'inoculation des souris avec 2,5.10⁶ CFU/souris est léthale à 50% au jour 5 et à 100% au jour 8 pour les souris du groupe placebo. Le premier décès intervient 3 jours après l'infection. Le score de morbidité décroît rapidement en dessous de 30/50 (25/50 au jour 2,5).

Le peptide pEM35 a été administré aux doses de 10 et 30 mg/kg/injection, à raison de 3 doses journalières pendant 4 jours (1h, 5h et 10h post-infection au jour 0 ; à 8h, 14h et 20h aux jours 1, 2 et 3) ; soit des doses journalières de 30 et 90 mg/kg.

Avec ce schéma d'administration, le pEM35 permet de retarder l'apparition du premier décès de 4 jours et demi pour les 2 doses. Au jour 8 post-infection, on observe un taux de survie, de 80% et 90%, respectivement, pour les souris traitées avec les doses de 30 et 90 mg/kg/jour. Les souris se maintiennent en bon état de santé jusqu'au jour 7, avec un score de morbidité qui se maintient au dessus de 40/50. Au jour 8, les scores diminuent à 30/50. Aucune différence majeure n'a été observée entre les groupes traités par le pEM35 à la dose faible de 30 mg/kg/jour et à celle forte de 90 mg/kg/jour.

Les courbes de survie relatives aux groupes traités par le pEM35 sont statistiquement différentes de la courbe relative au groupe placebo (p < 0.001 pour les deux doses).

### 6) Méthode - Modèle d'infection disséminée à Scedosporium inflatum.

Des souris Swiss OF1 de 22g sont infectées par voie intraveineuse (i.v.) avec une dose léthale de *Scedosporium inflatum*, (souche FSSP 7908 cultivée sur gélose Malt Agar pendant 7 jours à 37°C). La dose infectante est de 7.10⁶ spores par souris, injectée dans un volume de 100 µl par la veine latérale de la queue.

Les peptides pEM35 et pEM51 sont délivrés en continu avec des pompes osmotiques ALZET 1003D (débit : 0.97 µl/h ; volume : 93 µl : durée de perfusion : 4 jours) et 1007D (débit : 0.47 µl/h ; volume : 100 µl ; durée de perfusion : 8 jours et demi) implantées en intra-péritonéale.

Des groupes de 8 souris infectées sont traitées soit
a) par le placebo : NaCl 0.9% dans des pompes 1007D implantées en intra-péritonéale (i.p.);
b) non traitées ;
c) par le pEM51 délivré par des pompes 1007D en i.p. à une dose de 30 mg/kg pendant 8 jours correspondant à une concentration plasmatique théorique à l'équilibre de 0.3 µg/ml ;
d) par le pEM51 délivré par des pompes 1003D en i.p. à une dose de 60 mg/kg pendant 4 jours correspondant à une concentration plasmatique théorique à l'équilibre de 0.6 µg/ml ; e) par le pEM35 délivré par des pompes 1003D en i.p. à une dose de 35 mg/kg pendant 4 jours correspondant à une concentration plasmatique théorique à l'équilibre de 0.35 µg/ml.

### 7) Activité des analogues pEM35 et pEM51 délivrés en infusion continue dans un modèle de scédosporiose disséminée.

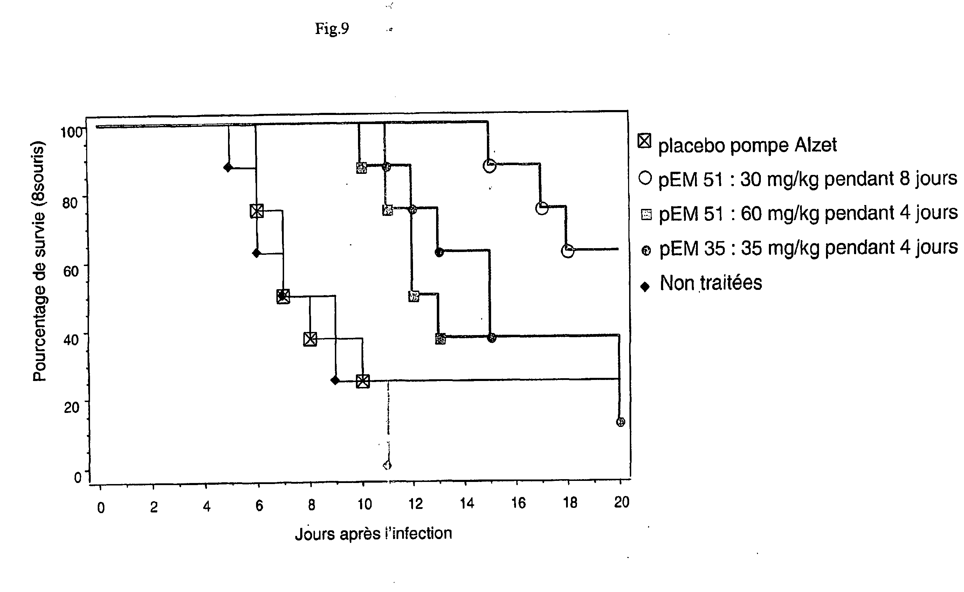

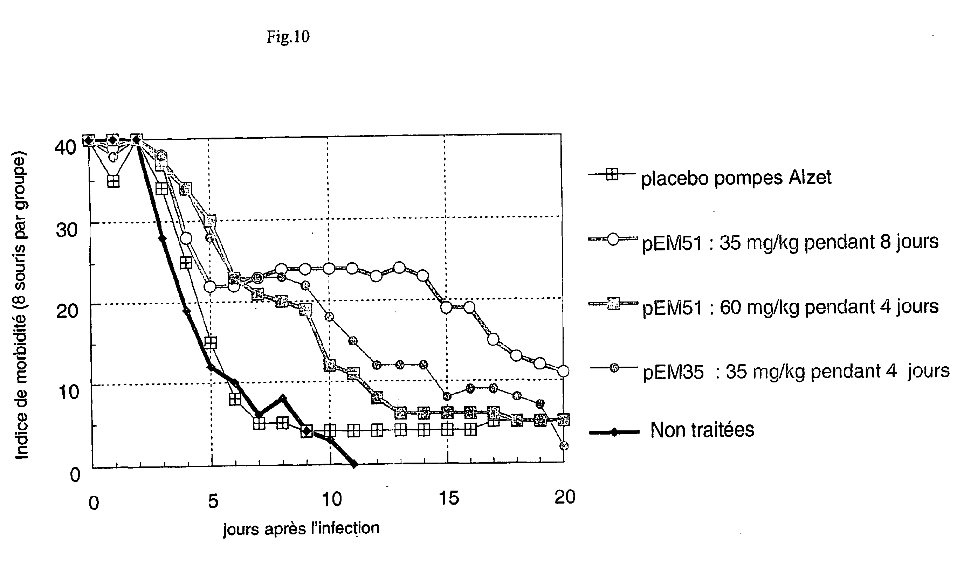

Les figures 9 et 10 en annexe représentent respectivement le pourcentage de survie et l'indice de morbidité (8 souris) par rapport aux jours post-infection.

Dans ce modèle de scédosporiose invasive, l'inoculation d'une dose de 7.10⁶ spores de *Scedosporium inflatum* est léthale à 50% au jour 7 après l'infection. Le premier décès intervient à 5 jours et à 6 jours après l'infection, respectivement, pour le groupe de souris témoin (infectées non traitées) et pour le groupe de souris placebo (infectées et implantées avec les pompes Alzet). On observe 100% de mortalité au jour 11 pour le groupe témoin et 75% de mortalité au jour 20 pour le groupe placebo. L'état de santé des souris se dégrade rapidement à partir du jour 3 après l'infection avec pour ces deux groupes, un score de morbidité de 28/40 et 34/40 au jour 3 et de 6/40 et 7/40 au jour 7 après l'infection. Des signes d'encéphalite apparaissent au jour 4 après l'infection.

Le traitement par le pEM51 à la dose de 30 mg/kg pendant 8 jours permet de retarder de 9 jours l'apparition du premier décès. Au jour 20, 5 souris sur 8 sont encore vivantes. L'indice de morbidité diminue à partir du jour 4 post-infection (28/40), correspondant à l'apparition de signe d'encéphalite, et se stabilise à 24/40 au jour 5 post-infection jusqu'au jour 14 post-infection. L'état des souris se dégrade ensuite progressivement, avec un indice de 11/40 au jour 20 post-infection. La courbe de mortalité est statistiquement différente de celles relatives aux groupes témoins et placebo. (logrank : p = 0.0027)

Le traitement par le pEM51 à la dose de 60 mg/kg pendant 4 jours permet de retarder de 4 jours l'apparition du premier décès. On observe 50% de mortalité au jour 12 post-infection, soit un délai de 5 jours par rapport aux groupes témoins et placebo. Au jour 20, 3 souris sur 8 sont encore vivantes. L'indice de morbidité diminue à partir du jour 5 post-infection (30/40), correspondant à l'apparition de signes d'encéphalite, et diminue progressivement à un indice de 6/40 au jour 14 post-infection. La courbe de mortalité est statistiquement différente de celles relatives aux groupes témoins et placebo. (logrank : p = 0.0176).

Le traitement par le pEM35 à la dose de 35 mg/kg pendant 4 jours permet de retarder de 5 jours l'apparition du premier décès. On observe 50% de mortalité au jour 15 post-infection, soit un délai de 8 jours par rapport aux groupes témoins et placebo. Au jour 20, 1 souris sur 8 est encore vivante. L'indice de morbidité diminue à partir du jour 5 post-infection (28/40), correspondant à l'apparition de signe d'encéphalite, et diminue progressivement à un indice de 8/40 au jour 15 post-infection. La courbe de mortalité est statistiquement différente de celles relatives aux groupes témoins et placebo. (logrank : p = 0.0177).

Dans ce modèle, le pEM51 administré à la dose de 30 mg/kg pendant 8 jours présente une très bonne efficacité thérapeutique en terme de survie. L'administration d'une dose 2 fois plus élevée (60mg/kg) sur une période 2 fois plus courte diminue nettement l'efficacité du pEM51. Toutefois, pendant les 4 premiers jours de traitement, le score de morbidité du groupe traité par la dose 30 mg/kg est sensiblement plus faible que celui du groupe traité par la dose 60 mg/kg. L'administration d'une dose de 60 mg/kg pendant 8 jours devrait donc encore améliorer l'efficacité thérapeutique du pEM51.

Le pEM35 à la dose de 35 mg/kg pendant 4 jours a démontré la même efficacité que le pEM51 à la dose de 60 mg/kg pendant 4 jours. L'activité thérapeutique du pEM35 dans ce modèle de Scédosporiose est donc au moins équivalente à celle du pEM51.

### 8) Etude de toxicité aiguë du pEM35 et pEM51 chez la souris.

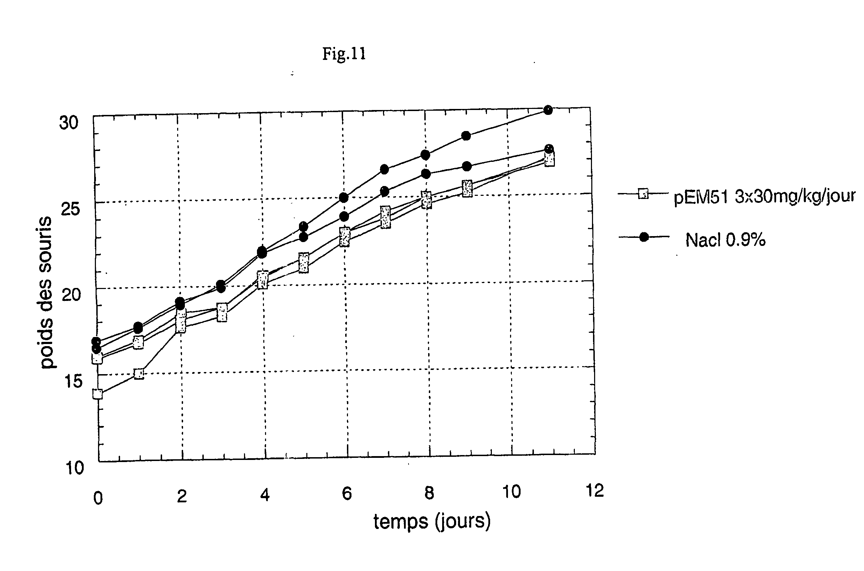

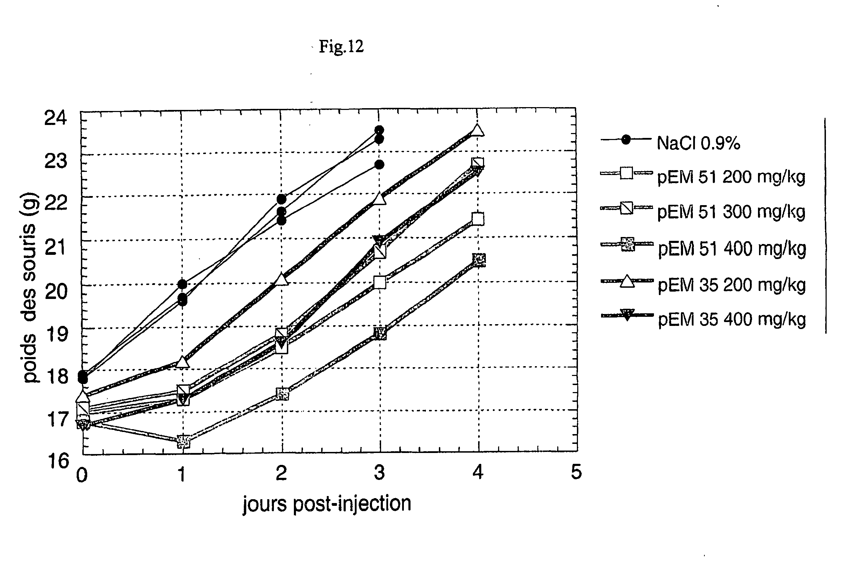

Les figures 11 et 12 en annexe représentent l'évolution pondérale de souris saines traitées en fonction du temps.

Au cours des essais d'efficacité thérapeutique chez la souris, aucune toxicité aiguë n'a été observée lors de l'administration en intraveineuse du pEM35 et du pEM51 dissout dans du NaCl 0.9%, en injections répétées à 30 minutes d'intervalle, de 3 fois 30 mg/kg pendant 3 jours.

L'évolution pondérale de souris saines traitées par 3 fois 30 mg/kg de pEM51 pendant 3 jours est semblable à celle des souris injectées avec du NaCl 0.9%.

La toxicité aiguë du pEM35 et du pEM51 en dose unique par voie intraveineuse, a été testée chez des souris mâles Swiss OF1 de 17-18 g à des doses de 200, 300 et 400 mg/kg. Les peptides sont solubilisés dans une solution de NaCl 0.9% ; le volume d'injection est de 150 µl, injecté en 45 secondes par la veine latérale de la queue.

Toutes les souris ont présenté une vasodilatation associée à une prostration. L'état des souris est redevenu normal 20 à 40 minutes après l'injection, en fonction de la dose.

Les courbes d'évolutions pondérales sur 4 jours montrent un léger retard de croissance le jour suivant l'injection, d'environ 1 g pour les souris ayant reçu le pEM35 aux doses de 200 et 400 mg/kg ou le pEM51 aux doses de 200 et 300 mg/kg ; et d'environ 2 g pour la souris ayant reçu le pEM51 à la dose de 400 mg/kg. L'évolution pondérale est ensuite normale pour toutes les souris.

### Exemple 7 : Spectre de l'activité antifongique de Ard1 et des analogues pEM31, pEM35, pEM46, pEM48 et pEM51.

### 1) Test de détection d'activité contre les champignons filamenteux.

L'activité antifongique a été détectée par un test d'inhibition de croissance en milieu liquide.

Les champignons filamenteux (*A. fumigatus, A. flavus* et *A. terreus*, don des Dr H. Koenig, Hopital Civil, Strasbourg ; et *S. prolificans* et *F. solani,* don des Dr. J. Meis et J. Mouton, Hopital Universitaire, Département Microbiologie, Nijmegen, Pays-Bas) sont ensemencés sur une gélose inclinée de Malt-Agar (Biomérieux) et incubés 7 jours à 37 °C.

Les spores sont ensuite récoltées avec 10 ml de milieu YPG contenant 0,05% de Tween 20 et filtrées à travers une gaze. Les spores sont centrifugées 10 min à 1700 rpm, le culot est repris dans du YPG (1 g de Yeast extract, 1 g de Peptone, 3 g de Glucose pour 1 1).

La suspension est comptée grâce à une cellule de comptage (Coverslide) et ajustée à 10⁴ spores/ml.

100 µl des dilutions de peptides (concentration de 50 à 0,097 µg/ml de peptide) sont déposées dans des plaques de microtitrations. 100 µl à 10⁴ spores/ml de champignons filamenteux soit 1000 spores sont ensuite ajoutées.

Les plaques de test sont incubées 48 h à 37°C.

La détermination des concentrations minimales inhibitrices (CMI) se fait par observation du pourcentage de recouvrement du puits. La CMI est établie pour un recouvrement de 50 % du puits.

### 2) Test de détection d'activité contre les levures.

Les levures du genre Candida (*C. albicans, C. glabrata, C. dubliensis, C. tropicalis, C. kefyr, C. krusei* et *C. parapsilosis ;* don du Dr H. Koenig, Hôpital Civil, Strasbourg), *C. albicans* résistants au fluconazole (n°245962, n°2332, n°246335 et n°3552 ; don des Dr. J. Meis et J. Mouton, Hopital Universitaire, département Microbiologie, Nijmegen Pays-Bas), et *Cryptoccocus neoformans* (don du Dr H. Koenig, Hôpital Civil, Strasbourg) sont ensemencées sur des géloses inclinées de Sabouraud-Cloramphénicol Agar (Biomérieux) et mises à incuber pendant 24 h à 30°C (*Candida sp*.) et pendant 72 h à 37°C (*Cryptoccocus neoformans*).

Quelques colonies de levures sont mises en suspension dans du milieu Sabouraud liquide (Biomérieux) afin d'obtenir une concentration finale de 0.1 DO à 600 nm correspondant à 2,5.10⁶ levures/ml.

La suspension de levures est ajustée à 5.10³ levures/ml dans du milieu Sabouraud.

100 µl des dilutions de peptides (concentration de 50 à 0,097 µg/ml de peptide) sont déposées dans des plaques de microtitrations. Après addition de 100 µl d'une suspension de levure à 5.10³ levures/ml soit 500 levures, les plaques de test sont incubées 24 h à 30°C (genre *Candida*) sous agitation lente ou 72h à 37°C (genre *Cryptoccus).*

La détermination des concentrations minimales inhibitrices (CMI) se fait par la mesure de l'absorbance à 600 nm à l'aide d'un spectrophotomètre lecteur de plaque de microtitration. La CMI est établie pour un pourcentage d'inhibition de pousse de 50 %.

### 3) Test de détection d'activité contre les phytopathogènes : Alternaria brassicola et Neurospora crassa.

100 µl des dilutions de peptides (concentration de 50 à 0,097 µg/ml de peptide) sont déposées dans des plaques de microtitrations.

Après addition de 100 µl de spores congelées à 10⁴ spores/ml d'*A. brassicola* et de *N. crassa* (don du Dr. Bullet, IBMC, Strasbourg), les plaques de test sont incubées 48 h à 30°C.

La détermination des concentrations minimales inhibitrices (CMI) se fait par observation du pourcentage de recouvrement du puits. La CMI est établie pour un recouvrement de 50 % du puits. Le tableau 3 ci-dessous représente les CMI de Ard1 et de ses analogues (µg/ml) contre les levures et les champignons filamenteux. Les tableaux 4 et 5 ci-dessous représentent les CMI des analogues pEM35 et pEM51 (µg/ml) respectivement contre les souches de levure *Candida albicans* résistantes au fluconazole et contre les champignons filamenteux.

**Tableau 3**

| Levures | Ard-1 | pEM 31 | pEM 48 | pEM 51 | pEM 46 | pEM 35 |
|---|---|---|---|---|---|---|
| *C. albicans* | 3,125-6,25 | 3,125-6,25 | 1,56 | 1,56-3,125 | 1,56-3,125 | 1,56 |
| *C. tropicalis* | 6,25-12,5 | 6,25 | 3,125 | 3,125 | 3,125 | 1,56 |
| *C. glabrata* | > 25 | > 25 | > 25 | > 25 | > 25 | > 25 |
| *C*. *parapsilosis* | 3,125-6,25 | 1,56 | 0,78 | 0,78-1,56 | 0,78-1,56 | 0,78-1,56 |
| *C. dubliensis* | 1,56-3,125 | 6,25 | 1,56-3,125 | 1,56-3,125 | 3,125 | 0,78-1,56 |
| *C. kefyr* | > 25 | > 25 | 25 | > 25 | > 25 | > 25 |
| *C. krusei* | 3,125 | 3,125 | 1,56 | 1,56 | 1,56 | 1,56 |
| *C. neoformans* | 12,5-25 | 12,5 | 3,125-6,25 | 1,56 | 6,25 | 6,25 |
| Chp. fltx | | | | | | |
| *A. fumigatus* | 12,5 | 6,25-12,5 | 6,25-12,5 | 6,25-12,5 | 3,125 | 6,25 |
| *A*. *flavus* | 6,25-12,5 | > 25 | 6,25-12,5 | 6,25 | 12,5-25 | 3,125 |
| *A. terreus* | 1,56-3,125 | 3,125-6,25 | 3,125-6,25 | 3,125 | 6,25 | 6,25 |
| *A. brassicola* | > 25 | > 25 | 12.5 | > 25 | 25 | > 25 |
| *N. crassa* | 0,097 | < 0,048 | 0,39 | 0,195 | 0,195 | < 0,048 |

**Tableau 4**

| *C. albicans* | pEM51 | pEM35 | ampho B | fluconazole | itraconazole |
|---|---|---|---|---|---|
| n° 245962 | 0,78-0,39 | 3,125-1,56 | 0,125 | > à 64 | 1-0,5 |
| n° 2332 | 1,56 | 1,56-0,79 | 0,25 | > à 64 | 0,5-0,25 |
| n° 246335 | 1,56-0,78 | 3,125-1,56 | 0,0625 | > à 64 | 0,5-0,25 |
| n° 3552 | 1,56-0,78 | 3,125-1,56 | 0,125-0,0625 | > à 64 | 1-0,5 |

**Tableau 5**

| Chp. Fltx | peptide | CMI (µg/ml) |
|---|---|---|
| FASF 5161 | pEM35 | 6,25-3,125 |
| A. *fumigatus* | pEM51 | 3,125-1,56 |
| | ampho B | 0,5 |
| FASF V02-31 | pEM35 | 12,5-6,25 |
| A. *fumigatus* | pEM51 | 12,5-6,25 |
| | ampho B | 1-0,5 |
| FSSP 7902 | pEM35 | 0,39-0,19 |
| S*. prolificans* | pEM51 | 0,19-0,09 |
| | ampho B | > 16 |
| FSSP 7908 | pEM35 | 0,19-0,09 |
| S. *prolificans* | pEM51 | 0,09-0,048 |
| | ampho B | > 16 |
| FFUS 8591 | pEM35 | 3,125-1,56 |
| *F. solani* | pEM51 | 0,78-0,39 |
| | ampho B | >16 |

### Exemple 8 : Cinétique de fongicidie des peptides pEM35 et pEM51 contre Candida albicans IHEM 8060.

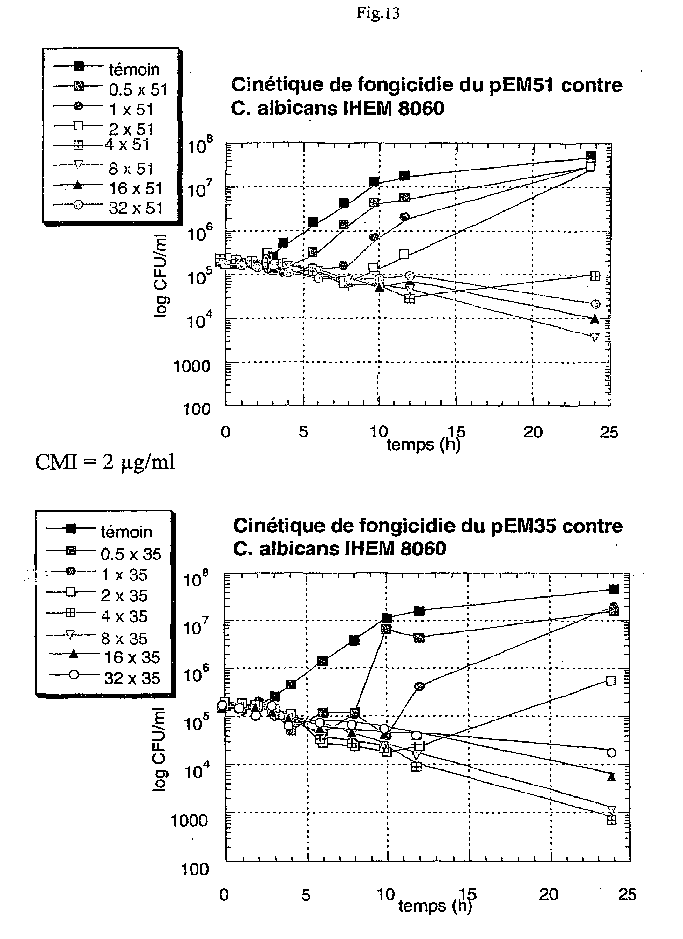

La figure 13 en annexe représente la cinétique de fongicidie des peptides pEM35 et pEM51 contre *Candida albicans* IHEM 8060.

Le test été effectué selon le protocole décrit par Klepser et al. (*Antimicrob Agents Chemother*, 1998 May, 42(5):1207-12 "Influence of test conditions on antifungal time-kill curve results: proposal for standardized methods"). Les souches de levures *Candida albicans* utilisées sont identiques à celles précédemment utilisées pour les tests de détection d'activité contre les levures (don du Dr. Koenig, Hôpital Civil, Strasbourg).

Les souches de levures sont ensemencées sur des géloses Sabouraud-Chloramphénicol et mises à incuber pendant 24 à 48 h à 30°C. Quelques colonies de levures sont mises en suspension dans 4 ml de milieu Sabouraud liquide (Biomérieux) puis incubées sous agitation pendant une nuit à 30°C.

La suspension de levures est ajustée à 1.10⁶⁻5.10⁶ levures/ml dans du Sabouraud frais. Une dilution 1 :10 est préparée par addition de 1 ml de la suspension de levures avec 9 ml de Sabouraud-Chloramphénicol (Biomérieux) contenant ou non (contrôle) une quantité définie de peptide pEM35 ou pEM51. La concentration de levures dans l'inoculum de départ est ainsi de 1.10⁵-5.10⁵ levures/ml.

Les peptides pEM35 et pEM51 sont testés sur une gamme de concentration s'échelonnant de 1 µg/ml à 64 µg/ml. Chacune des solutions est incubée à 35°C. A des temps prédéterminés (0, 1, 2, 3, 4, 6, 8, 10 et 24 h), un échantillon de 100 µl de chacune des solutions est prélevé et dilué en série 10 fois dans de l'eau stérile. Une aliquote de 30 µl est ensuite étalée sur boîtes de gélose Sabouraud (Biomérieux) afin de décompter les colonies. Lorsque le nombre de colonies est, selon estimation, inférieur à 1000 levures/ml, un échantillon de 30 µl est prélevé directement à partir de la solution test et étalé sur boîtes de gélose Sabouraud (Biomérieux) sans dilution préalable. Les boîtes sont incubées pendant 24 à 48 heures à 35°C.

Un test contrôle amphotéricine B (concentration correspondant à 1 fois et 16 fois la CMI) est réalisé en parallèle selon le protocole décrit par Klepser et al. (*Antimicrob Agents Chemother* 1997 June; 41(6):1392-1395, "Antifungal pharmacodynamic characteristics of fluconazole and Amphotericin B tested against *Candida albicans*").

Le seuil minimal de détection du nombre de levures/ml est déterminé par préparation d'une suspension de levure *Candida albicans* dans de l'eau stérile avec le peptide pEM35 ou pEM51 puis ajustement à 0,5 turbidité standard Mc Farland (concentration 1.10⁶-5.10⁶ levures/ml). Des dilutions dans l'eau stérile sont réalisées de façon à obtenir 3 suspensions de concentration respective 100, 50 et 30 levures/ml. 30 µl de chaque suspension sont prélevés et étalés sur boîtes de gélose Sabouraud (Biomérieux) afin de décompter les colonies. Les boîtes sont incubées pendant 24 à 48 heures à 35°C.

Les valeurs décomptées (log₁₀ levures/ml) sont reportées sur l'échelle de temps prédéfinie pour chacune des concentrations de peptide pEM35 et pEM51 testées.

## Revendications

1. Peptide dérivé de l'héliomicine, **caractérisé en ce que** sa séquence en acides aminés correspond à celle de l'héliomicine dans laquelle les régions hydrophobes et chargées présentent une ou plusieurs mutations et **en ce que** ledit peptide est choisi parmi les séquences en acides aminés suivantes :

2. Peptide dérivé de l'héliomicine, **caractérisé en ce que** sa séquence en acides aminés correspond à celle de l'héliomicine dans laquelle les régions hydrophobes et chargées présentent une ou plusieurs mutations et **en ce que** ledit peptide est choisi parmi les séquences en acides aminés suivantes :

3. Composition antifongique et/ou antibactérienne, **caractérisé en ce qu'**elle comprend à titre d'agent actif au moins un peptide selon l'une quelconque des revendications 1 ou 2, avantageusement associé dans ladite composition avec un véhicule acceptable.

4. Composition selon la revendication 3 pour être utilisée chez l'homme ou l'animal.

5. Composition selon la revendication 3 pour être utilisée chez les plantes.

6. Séquence d'acide nucléique, **caractérisée en ce qu'**elle code pour un peptide selon l'une quelconque des revendications 1 ou 2.

7. Vecteur d'expression, **caractérisé en ce qu'**il comprend une séquence d'acide nucléique selon la revendication 6.

8. Cellule végétale, **caractérisée en ce qu'**elle comprend une séquence d'acide nucléique selon la revendication 6.

9. Plante résistante aux maladies, **caractérisée en ce qu'**elle comprend une séquence d'acide nucléique selon la revendication 6 et qu'elle exprime un peptide selon l'une quelconque des revendications 1 ou 2.

## Patentansprüche

1. Peptid deriviert aus dem Heliomicin, **dadurch gekennzeichnet, dass** seine Sequenz von Aminosäuren derjenigen vom Heliomicin gleichkommt, in der die hydrophoben und belasteten Regionen eine oder mehrere Mutationen aufweisen und **dadurch gekennzeichnet, dass** das genannte Peptid zwischen den nachstehenden Sequenzen von Aminosäuren gewählt wird:

2. Peptid deriviert aus dem Heliomicin, **dadurch gekennzeichnet, dass** seine Sequenz von Aminosäuren derjenigen vom Heliomicin gleichkommt, in der die hydrophoben und belasteten Regionen eine oder mehrere Mutationen aufweisen und **dadurch gekennzeichnet, dass** das genannte Peptid zwischen den nachstehenden Sequenzen von Aminosäuren gewählt wird:

3. Antifungische und/oder antibakterielle Verbindung, **dadurch gekennzeichnet, dass** sie als Wirkstoff zumindest ein Peptid nach einem der Ansprüche 1 oder 2 umfasst, vorteilhaft assoziiert in der genannten Verbindung mit einem annehmbaren Fahrzeug.

4. Verbindung nach Anspruch 3, um beim Menschen oder Tier eingesetzt zu werden.

5. Verbindung nach Anspruch 3, um bei den Pflanzen eingesetzt zu werden.

6. Sequenz von Nucleinsäure, **dadurch gekennzeichnet, dass** sie für ein Peptid nach einem der Ansprüche 1 oder 2 kodiert.

7. Ausdrucksvektor, **dadurch gekennzeichnet, dass** er eine Sequenz von Nucleinsäure nach Anspruch 6 umfasst.

8. Pflanzenzelle, **dadurch gekennzeichnet, dass** sie eine Sequenz von Nucleinsäure nach Anspruch 6 umfasst.

9. Krankheitsresistente Pflanze **dadurch gekennzeichnet, dass** sie eine Sequenz von Nucleinsäure nach Anspruch 6 umfasst und dass sie ein Peptid nach einem der Ansprüche 1 oder 2 ausdrückt.

## Claims

1. Peptide derived from heliomicin, **characterised in that** its amino acids sequence corresponds to the amino acids sequence of heliomicin in which the hydrophobic and charged regions have one or several mutations and **in that** the said peptide is chosen from among the following amino acid sequences:

2. Peptide derived from heliomicin, **characterised in that** its amino acids sequence corresponds to the amino acids sequence of heliomicin in which the hydrophobic and charged regions have one or several mutations and **in that** the said peptide is chosen from among the following amino acid sequences:

3. Antifungal and / or antibacterial composition, **characterised in that** it comprises at least one peptide according to either of claims 1 or 2 as an active agent, advantageously associated with an acceptable vehicle in the said composition.

4. Composition according to claim 3 for use in man or animals.

5. Composition according to claim 3 for use in plants.

6. Nucleic acid sequence, **characterised in that** it is coding for a peptide according to either of claims 1 or 2.

7. Expression vector, **characterised in that** it comprises a nucleic acid sequence according to claim 6.

8. Plant cell, **characterised in that** it comprises a nucleic acid sequence according to claim 6.

9. Plant resistant to diseases, **characterised in that** it comprises a nucleic acid sequence according to claim 6 and **in that** it expresses a peptide according to either of claims 1 or 2.


### LISTE DE SEQUENCES

<110> ENTOMED S.A.
<120> PEPTIDES ANTIFONGIQUES ET/OU ANTIBACTERIENS, LEURS PREPARATIONS ET LES COMPOSITIONS LES CONTENANT.
<130> (9800+8219)PCT-Hélio-juillet 2001
<140> XXXXXXXX
   <141> 2001-07-13
<150> FR00/11949
   <151> 2000-09-19
<150> FR00/09248
   <151> 2000-07-13
<160> 96
<170> PatentIn Ver. 2.1
<210> 1
   <211> 44
   <212> PRT
   <213> Séquence artificielle
<220>
   <223> Description de la séquence artificielle: héliomicine
<400> 1
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 2
   <211> 44
   <212> PRT
   <213> Séquence artificielle
<220>
   <223> Description de la séquence artificielle: Ard1, peptide homologue de l'héliomicine
<400> 2
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 3
   <211> 44
   <212> PRT
   <213> Séquence artificielle
<220>
   <223> Description de la séquence artificielle: peptide dérivé de l'héliomicine
<220>
   <223> peptide pEM37
<400> 3
   Asn Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 4
   <211> 44
   <212> PRT
   <213> Séquence artificielle
<220>
   <223> Description de la séquence artificielle: peptide dérivé de l'héliomicine
<220>
   <223> peptide pEM38
<400> 4
   Asp Lys Leu Ile Gly Thr Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 5
   <211> 44
   <212> PRT
   <213> Séquence artificielle
<220>
   <223> Description de la séquence artificielle: peptide dérivé de l'héliomicine
<220>
   <223> peptide pEM43
<400> 5
   Asp Lys Leu Ile Gly Ser Cys Val Trp Gly Ala Val Asn Tyr Thr Thr
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 6
   <211> 44
   <212> PRT
   <213> Séquence artificielle
<220>
   <223> Description de la séquence artificielle:peptide dérivé de l'héliomicine
<220>
   <223> peptide pEM42
<400> 6
   Asp Lys Leu Ile Gly Ser Cys Val Trp Gly Ala Val Asn Tyr Thr Arg
    1               5                  10                  15
   Asp Cys Asn Gly Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 7
   <211> 44
   <212> PRT
   <213> Séquence artificielle
<220>
   <223> Description de la séquence artificielle:peptide dérivé de l'héliomicine
<220>
   <223> peptide pEM44
<400> 7
   Asp Lys Leu Ile Gly Ser Cys Val Trp Gly Ala Val Asn Tyr Thr Ser
    1               5                  10                  15
   Asp Cys Arg Gly Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 8
   <211> 44
   <212> PRT
   <213> Séquence artificielle
<220>
   <223> Description de la séquence artificielle:peptide dérivé de l'héliomicine
<220>
   <223> peptide pEM22
<400> 8
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ile Asn Val Asn Cys Trp Cys Glu Thr
   35 40
<210> 9
   <211> 44
   <212> PRT
   <213> Séquence artificielle
<220>
   <223> Description de la séquence artificielle: peptide dérivé de l'héliomicine
<220>
   <223> peptide pEM23
<400> 9
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 10
   <211> 44
   <212> PRT
   <213> Séquence artificielle
<220>
   <223> Description de la séquence artificielle: peptide dérivé de l'héliomicine
<220>
   <223> peptide pEM25
<400> 10
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Leu Asn Val Asn Cys Trp Cys Glu Thr
           35                  40
<210> 11
   <211> 44
   <212> PRT
   <213> Séquence artificielle
<220>
   <223> Description de la séquence artificielle: peptide dérivé de l'héliomicine
<220>
   <223> peptide pEM24
<400> 11
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Leu Asn Ile Asn Cys Trp Cys Glu Thr
   35                  40
<210> 12
   <211> 44
   <212> PRT
   <213> Séquence artificielle
<220>
   <223> Description de la séquence artificielle: peptide dérivé de l'héliomicine
<220>
   <223> peptide pEM7
<400> 12
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Ala Asn Val Asn Cys Trp Cys Glu Arg
           35                  40
<210> 13
   <211> 44
   <212> PRT
   <213> Séquence artificielle
<220>
   <223> Description de la séquence artificielle: peptide dérivé de l'héliomicine
<220>
   <223> peptide pEM21
<400> 13
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Ala Asn Val Asn Cys Trp Cys Gln Thr
           35                  40
<210> 14
   <211> 44
   <212> PRT
   <213> Séquence artificielle
<220>
   <223> Description de la séquence artificielle: peptide dérivé de l'héliomicine
<220>
   <223> peptide pEM39
<400> 14
   Asn Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Ala Asn Val Asn Cys Trp Cys Gln Thr
   35 40
<210> 15
   <211> 44
   <212> PRT
   <213> Séquence artificielle
<220>
   <223> Description de la séquence artificielle: peptide dérivé de l'héliomicine
<220>
   <223> peptide pEM61
<400> 15
   Asn Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Leu Asn Val Asn Cys Trp Cys Gln Thr
           35                  40
<210> 16
   <211> 44
   <212> PRT
   <213> Séquence artificielle
<220>
   <223> Description de la séquence artificielle: peptide dérivé de l'héliomicine
<220>
   <223> peptide pEM62
<400> 16
   Asn Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Ile Asn Val Asn Cys Trp Cys Gln Thr
           35                  40
<210> 17
   <211> 44
   <212> PRT
   <213> Séquence artificielle
<220>
   <223> Description de la séquence artificielle: peptide dérivé de l'héliomicine
<220>
   <223> peptide pEM40
<400> 17
   Asn Lys Leu Ile Gly Ser Cys Val Trp Gly Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Glu Thr
                       35                  40
<210> 18
   <211> 44
   <212> PRT
   <213> Séquence artificielle
<220>
   <223> Description de la séquence artificielle: peptide dérivé de l'héliomicine
<220>
   <223> peptide pEM50
<400> 18
   Asp Lys Leu Ile Gly Ser Cys Val Trp Gly Ala Val Asn Tyr Thr Arg
    1               5                  10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 19
   <211> 44
   <212> PRT
   <213> Séquence artificielle
<220>
   <223> Description de la séquence artificielle: peptide dérivé de l'héliomicine
<220>
   <223> peptide pEM56
<400> 19
   Asp Lys Leu Ile Gly Ser Cys Val Trp Leu Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ala Asn Val Asn Cys Trp Cys Glu Thr
   35 40
<210> 20
   <211> 44
   <212> PRT
   <213> Séquence artificielle
<220>
   <223> Description de la séquence artificielle:peptide dérivé de l'héliomicine
<220>
   <223> peptide pEM52
<400> 20
   Asp Lys Leu Ile Gly Ser Cys Val Trp Gly Ala Val Asn Tyr Thr Ser
   1 5 10 15
   Arg Cys Asn Ala Glu Cys Lys Arg Arg Gly Tyr Lys Gly Gly His Cys
   20 25 30
   Gly Ser Phe Ala Asn Val Asn Cys Trp Cys Glu Thr
   35 40
<210> 21
   <211> 44
   <212> PRT
   <213> Séquence artificielle
<220>
   <223> Description de la séquence artificielle: peptide dérivé de l'héliomicine
<220>
   <223> peptide pEM51
<400> 21
   Asp Lys Leu Ile Gly Ser Cys Val Trp Gly Ala Val Asn Tyr Thr Ser
   1 5 10 15
   Asn Cys Arg Ala Glu Cys Lys Arg Arg Gly Tyr Lys Gly Gly His Cys
   20 25 30
   Gly Ser Phe Ala Asn Val Asn Cys Trp Cys Glu Thr
   35 40
<210> 22
   <211> 44
   <212> PRT
   <213> Séquence artificielle
<220>
   <223> Description de la séquence artificielle: peptide dérivé de l'héliomicine
<220>
   <223> peptide pEM32
<400> 22
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ile Asn Val Asn Cys Trp Cys Glu Thr
   35 40
<210> 23
   <211> 44
   <212> PRT
   <213> Séquence artificielle
<220>
   <223> Description de la séquence artificielle: peptide dérivé de l'héliomicine
<220>
   <223> peptide pEM33
<400> 23
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ala Asn Ile Asn Cys Trp Cys Glu Thr
   35 40
<210> 24
   <211> 44
   <212> PRT
   <213> Séquence artificielle
<220>
   <223> Description de la séquence artificielle: peptide dérivé de l'héliomicine
<220>
   <223> peptide pEM34
<400> 24
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Leu Asn Ile Asn Cys Trp Cys Glu Thr
           35                  40
<210> 25
   <211> 44
   <212> PRT
   <213> Séquence artificielle
<220>
   <223> Description de la séquence artificielle: peptide dérivé de l'héliomicine
<220>
   <223> peptide pEM35
<400> 25
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Leu Asn Val Asn Cys Trp Cys Glu Thr
           35                  40
<210> 26
   <211> 44
   <212> PRT
   <213> Séquence artificielle
<220>
   <223> Description de la séquence artificielle: peptide dérivé de l'héliomicine
<220>
   <223> peptide pEM31
<400> 26
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Gln Thr
   35                  40
<210> 27
   <211> 44
   <212> PRT
   <213> Séquence artificielle
<220>
   <223> Description de la séquence artificielle: peptide dérivé de l'héliomicine
<220>
   <223> peptide pEM30
<400> 27
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Glu Arg
           35                  40
<210> 28
   <211> 44
   <212> PRT
   <213> Séquence artificielle
<220>
   <223> Description de la séquence artificielle: peptide dérivé de l'héliomicine
<220>
   <223> peptide pEM46
<400> 28
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Leu Asn Val Asn Cys Trp Cys Gln Thr
   35 40
<210> 29
   <211> 44
   <212> PRT
   <213> Séquence artificielle
<220>
   <223> Description de la séquence artificielle: peptide dérivé de l'héliomicine
<220>
   <223> peptide pEM47
<400> 29
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ile Asn Val Asn Cys Trp Cys Gln Thr
   35 40
<210> 30
   <211> 44
   <212> PRT
   <213> Séquence artificielle
<220>
   <223> Description de la séquence artificielle: peptide dérivé de l'héliomicine
<220>
   <223> peptide pEM48
<400> 30
   Asp Lys Leu Ile Gly Ser Cys Val Trp Gly Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Leu Asn Val Asn Cys Trp Cys Glu Arg
           35                  40
<210> 31
   <211> 44
   <212> PRT
   <213> Séquence artificielle
<220>
   <223> Description de la séquence artificielle: peptide dérivé de l'héliomicine
<220>
   <223> peptide pEM49
<400> 31
   Asp Lys Leu Ile Gly Ser Cys Val Trp Gly Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Ile Asn Val Asn Cys Trp Cys Glu Arg
           35                  40
<210> 32
   <211> 44
   <212> PRT
   <213> Séquence artificielle
<220>
   <223> Description de la séquence artificielle: peptide dérivé de l'héliomicine
<220>
   <223> peptide pEM54
<400> 32
   Asp Lys Leu Ile Gly Ser Cys Val Trp Leu Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Leu Asn Val Asn Cys Trp Cys Glu Thr
   35 40
<210> 33
   <211> 44
   <212> PRT
   <213> Séquence artificielle
<220>
   <223> Description de la séquence artificielle: peptide dérivé de l'héliomicine
<220>
   <223> peptide pEM57
<400> 33
   Asp Lys Leu Ile Gly Ser Cys Val Trp Leu Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Gln Thr
           35                  40
<210> 34
   <211> 44
   <212> PRT
   <213> Séquence artificielle
<220>
   <223> Description de la séquence artificielle: peptide dérivé de l'héliomicine
<220>
   <223> peptide pEM55
<400> 34
   Asp Lys Leu Ile Gly Ser Cys Val Trp Leu Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Leu Asn Val Asn Cys Trp Cys Gln Thr
           35                  40
<210> 35
   <211> 44
   <212> PRT
   <213> Séquence artificielle
<220>
   <223> Description de la séquence artificielle: peptide dérivé de l'héliomicine
<220>
   <223> peptide pEM37
<400> 35
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 36
   <211> 44
   <212> PRT
   <213> Séquence artificielle
<220>
   <223> Description de la séquence artificielle: peptide dérivé de l'héliomicine
<220>
   <223> peptide pEM38
<400> 36
   Asp Lys Leu Ile Gly Thr Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 37
   <211> 44
   <212> PRT
   <213> Séquence artificielle
<220>
   <223> Description de la séquence artificielle: peptide dérivé de l'héliomicine
<220>
   <223> peptide pEM45
<400> 37
   Asp Lys Leu Ile Gly Ser Cys Val Trp Gly Ala Val Arg Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 38
   <211> 44
   <212> PRT
   <213> Séquence artificielle
<220>
   <223> Description de la séquence artificielle: peptide dérivé de l'héliomicine
<220>
   <223> peptide pEM42
<400> 38
   Asp Lys Leu Ile Gly Ser Cys Val Trp Gly Ala Val Asn Tyr Thr Arg
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 39
   <211> 44
   <212> PRT
   <213> Séquence artificielle
<220>
   <223> Description de la séquence artificielle: peptide dérivé de l'héliomicine
<220>
   <223> peptide pEM44
<400> 39
   Asp Lys Leu Ile Gly Ser Cys Val Trp Gly Ala Val Asn Tyr Thr Ser
   1 5 10 15
   Asp Cys Arg Gly Glu Cys Lys Arg Arg Gly Tyr Lys Gly Gly His Cys
   20 25 30
   Gly Ser Phe Ala Asn Val Asn Cys Trp Cys Glu Thr
   35 40
<210> 40
   <211> 44
   <212> PRT
   <213> Séquence artificielle
<220>
   <223> Description de la séquence artificielle: peptide dérivé de l'héliomicine
<220>
   <223> peptide pEM22
<400> 40
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ile Asn Val Asn Cys Trp Cys Glu Thr
   35 40
<210> 41
   <211> 44
   <212> PRT
   <213> Séquence artificielle
<220>
   <223> Description de la séquence artificielle: peptide dérivé de l'héliomicine
<220>
   <223> peptide pEM23
<400> 41
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Ile Asn Cys Trp Cys Glu Thr
   35 40
<210> 42
   <211> 44
   <212> PRT
   <213> Séquence artificielle
<220>
   <223> Description de la séquence artificielle: peptide dérivé de l'héliomicine
<220>
   <223> peptide pEM25
<400> 42
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Leu Asn Val Asn Cys Trp Cys Glu Thr
           35                  40
<210> 43
   <211> 44
   <212> PRT
   <213> Séquence artificielle
<220>
   <223> Description de la séquence artificielle: peptide dérivé de l'héliomicine
<220>
   <223> peptide pEM24
<400> 43
   Asp Lys Leu Ile Gly Ser Cys Val Trp Gly Ala Val Asn Tyr Thr Ser
   1               5                   10                  15
   Asp Cys Asn Gly Glu Cys Lys Arg Arg Gly Tyr Lys Gly Gly His Cys
               20                  25                  30
   Gly Ser Phe Leu Asn Ile Asn Cys Trp Cys Glu Thr
           35                  40
<210> 44
   <211> 44
   <212> PRT
   <213> Séquence artificielle
<220>
   <223> Description de la séquence artificielle: peptide dérivé de l'héliomicine
<220>
   <223> peptide pEM7
<400> 44
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Glu Arg
   35 40
<210> 45
   <211> 44
   <212> PRT
   <213> Séquence artificielle
<220>
   <223> Description de la séquence artificielle: peptide dérivé de l'héliomicine
<220>
   <223> peptide pEM21
<400> 45
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Gln Thr
   35 40
<210> 46
   <211> 44
   <212> PRT
   <213> Séquence artificielle
<220>
   <223> Description de la séquence artificielle: peptide dérivé de l'héliomicine
<220>
   <223> peptide pEM39
<400> 46
   Asp Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Val Asn Cys Trp Cys Gln Thr
   35 40
<210> 47
   <211> 44
   <212> PRT
   <213> Séquence artificielle
<220>
   <223> Description de la séquence artificielle: peptide dérivé de l'héliomicine
<220>
   <223> peptide pEM61
<400> 47
   Asn Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Leu Asn Cys Trp Cys Gln Thr
   35 40
<210> 48
   <211> 44
   <212> PRT
   <213> Séquence artificielle
<220>
   <223> Description de la séquence artificielle: peptide dérivé de l'héliomicine
<220>
   <223> peptide pEM62
<400> 48
   Asn Lys Leu Ile Gly Ser Cys Val Trp Gly Ala Val Asn Tyr Thr Ser
   1 5 10 15
   Asp Cys Asn Gly Glu Cys Lys Arg Arg Gly Tyr Lys Gly Gly His Cys
   20 25 30
   Gly Ser Phe Ala Asn Ile Asn Cys Trp Cys Gln Thr
   35 40
<210> 49
   <211> 44
   <212> PRT
   <213> Séquence artificielle
<220>
   <223> Description de la séquence artificielle: peptide dérivé de l'héliomicine
<220>
   <223> peptide pEM50
<400> 49
   Asn Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 50
   <211> 44
   <212> PRT
   <213> Séquence artificielle
<220>
   <223> Description de la séquence artificielle: peptide dérivé de l'héliomicine
<220>
   <223> peptide pEM56
<400> 50
   Asp Lys Leu Ile Gly Ser Cys Val Trp Gly Ala Val Asn Tyr Thr Arg
   1               5                   10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Glu Thr
                           35                  40
<210> 51
   <211> 44
   <212> PRT
   <213> Séquence artificielle
<220>
   <223> Description de la séquence artificielle: peptide dérivé de l'héliomicine
<220>
   <223> peptide pEM56
<400> 51
   Asp Lys Leu Ile Gly Ser Cys Val Trp Leu Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ala Asn Val Asn Cys Trp Cys Glu Thr
   35 40
<210> 52
   <211> 44
   <212> PRT
   <213> Séquence artificielle
<220>
   <223> Description de la séquence artificielle: peptide dérivé de l'héliomicine
<220>
   <223> peptide pEM52
<400> 52
   Asp Lys Leu Ile Gly Ser Cys Val Trp Gly Ala Val Asn Tyr Thr Ser
   1 5 10 15
   Arg Cys Asn Ala Glu Cys Lys Arg Arg Gly Tyr Lys Gly Gly His Cys
   20 25 30
   Gly Ser Phe Ala Asn Val Asn Cys Trp Cys Glu Thr
   35 40
<210> 53
   <211> 44
   <212> PRT
   <213> Séquence artificielle
<220>
   <223> Description de la séquence artificielle: peptide dérivé de l'héliomicine
<220>
   <223> peptide pEM51
<400> 53
   Asp Lys Leu Ile Gly Ser Cys Val Trp Gly Ala Val Asn Tyr Thr Ser
   1 5 10 15
   Asn Cys Arg Ala Glu Cys Lys Arg Arg Gly Tyr Lys Gly Gly His Cys
   20 25 30
   Gly Ser Phe Ala Asn Val Asn Cys Trp Cys Glu Thr
   35 40
<210> 54
   <211> 44
   <212> PRT
   <213> Séquence artificielle
<220>
   <223> Description de la séquence artificielle: peptide dérivé de l'héliomicine
<220>
   <223> peptide pEM32
<400> 54
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ile Asn Val Asn Cys Trp Cys Glu Thr
   35 40
<210> 55
   <211> 44
   <212> PRT
   <213> Séquence artificielle
<220>
   <223> Description de la séquence artificielle: peptide dérivé de l'héliomicine
<220>
   <223> peptide pEM33
<400> 55
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ala Asn Ile Asn Cys Trp Cys Glu Thr
   35 40
<210> 56
   <211> 44
   <212> PRT
   <213> Séquence artificielle
<220>
   <223> Description de la séquence artificielle: peptide dérivé de l'héliomicine
<220>
   <223> peptide pEM34
<400> 56
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Leu Asn Ile Asn Cys Trp Cys Glu Thr
                         35                  40
<210> 57
   <211> 44
   <212> PRT
   <213> Séquence artificielle
<220>
   <223> Description de la séquence artificielle: peptide dérivé de l'héliomicine
<220>
   <223> peptide pEM35
<400> 57
   Asp Lys Leu Ile Gly Ser Cys Val Trp Gly Ala Val Asn Tyr Thr Ser
   1               5                   10                  15
   Asn Cys Asn Ala Glu Cys Lys Arg Arg Gly Tyr Lys Gly Gly His Cys
               20                  25                  30
   Gly Ser Phe Leu Asn Val Asn Cys Trp Cys Glu Thr
           35                  40
<210> 58
   <211> 44
   <212> PRT
   <213> Séquence artificielle
<220>
   <223> Description de la séquence artificielle: peptide dérivé de l'héliomicine
<220>
   <223> peptide pEM31
<400> 58
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ala Asn Val Asn Cys Trp Cys Gln Thr
   35 40
<210> 59
   <211> 44
   <212> PRT
   <213> Séquence artificielle
<220>
   <223> Description de la séquence artificielle: peptide dérivé de l'héliomicine
<220>
   <223> peptide pEM30
<400> 59
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ala Asn Val Asn Cys Trp Cys Glu Arg
   35 40
<210> 60
   <211> 44
   <212> PRT
   <213> Séquence artificielle
<220>
   <223> Description de la séquence artificielle: peptide dérivé de l'héliomicine
<220>
   <223> peptide pEM46
<400> 60
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Leu Asn Val Asn Cys Trp Cys Gln Thr
   35 40
<210> 61
   <211> 44
   <212> PRT
   <213> Séquence artificielle
<220>
   <223> Description de la séquence artificielle: peptide dérivé de l'héliomicine
<220>
   <223> peptide pEM47
<400> 61
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Ile Asn Val Asn Cys Trp Cys Gln Thr
   35 40
<210> 62
   <211> 44
   <212> PRT
   <213> séquence artificielle
<220>
   <223> Description de la séquence artificielle: peptide dérivé de l'héliomicine
<220>
   <223> peptide pEM48
<400> 62
   Asp Lys Leu Ile Gly Ser Cys Val Trp Gly Ala Val Asn Tyr Thr Ser
   1 5 10 15
   Asn Cys Asn Ala Glu Cys Lys Arg Arg Gly Tyr Lys Gly Gly His Cys
   20 25 30
   Gly Ser Phe Leu Asn Val Asn Cys Trp Cys Glu Arg
   35 40
<210> 63
   <211> 44
   <212> PRT
   <213> Séquence artificielle
<220>
   <223> Description de la séquence artificielle: peptide dérivé de l'héliomicine
<220>
   <223> peptide pEM49
<400> 63
   Asp Lys Leu Ile Gly Ser Cys Val Trp Gly Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Ile Asn Val Asn Cys Trp Cys Glu Arg
           35                  40
<210> 64
   <211> 44
   <212> PRT
   <213> Séquence artificielle
<220>
   <223> Description de la séquence artificielle: peptide dérivé de l'héliomicine
<220>
   <223> peptide pEM54
<400> 64
   Asp Lys Leu Ile Gly Ser Cys Val Trp Leu Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Leu Asn Val Asn Cys Trp Cys Glu Thr
           35                  40
<210> 65
   <211> 44
   <212> PRT
   <213> Séquence artificielle
<220>
   <223> Description de la séquence artificielle: peptide dérivé de l'héliomicine
<220>
   <223> peptide pEM54
<400> 65
   Asp Lys Leu Ile Gly Ser Cys Val Trp Leu Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Gln Thr
                       35                  40
<210> 66
   <211> 44
   <212> PRT
   <213> Séquence artificielle
<220>
   <223> Description de la séquence artificielle: peptide dérivé de l'héliomicine
<220>
   <223> peptide pEM55
<400> 66
   Asp Lys Leu Ile Gly Ser Cys Val Trp Leu Ala Val Asn Tyr Thr Ser
    1               5                  10                  15
   Asn Cys Asn Ala Glu Cys Lys Arg Arg Gly Tyr Lys Gly Gly His Cys
               20                  25                  30
   Gly Ser Phe Ala Asn Val Asn Cys Trp Cys Gln Thr
           35                  40
<210> 67
   <211> 26
   <212> ADN
   <213> Séquence artificielle
<220>
   <223> Description de la séquence artificielle: oligonucléotide EM72
<400> 67
   gtaaatgcat gtatactaaa ctcaca                                      26
<210> 68
   <211> 55
   <212> ADN
   <213> Séquence artificielle
<220>
   <223> Description de la séquence artificielle: oligonucléotide EM89
<400> 68
   ttttttccgc ggcgcttgca ctcggcgttg cagttactag tgtagttgac ggcgc 55
<210> 69
   <211> 47
   <212> ADN
   <213> Séquence artificielle
<220>
   <223> Description de la séquence artificielle: oligonucléotide EM89 brin complémentaire
<400> 69
   cgccgcgaac gtgagccgca acgtcaatga tcacatcaac tgccgcg 47
<210> 70
   <211> 15
   <212> PRT
   <213> Séquence artificielle
<220>
   <223> Description de la séquence artificielle: peptide muté analogue de Ard1
<400> 70
   Arg Arg Lys Cys Glu Ala Asn Cys Asn Ser Thr Tyr Asn Val Ala
   1 5 10 15
<210> 71
   <211> 20
   <212> ADN
   <213> Séquence artificielle
<220>
   <223> Description de la séquence artificielle: oligonucléotide EM25
<400> 71
   gatccactcg agtgctagcg 20
<210> 72
   <211> 20
   <212> ADN
   <213> Séquence artificielle
<220>
   <223> Description de la séquence artificielle: oligonucléotide EM26
<400> 72
   tcgacgctag cactcgagtg 20
<210> 73
   <211> 42
   <212> ADN
   <213> Séquence artificielle
<220>
   <223> Description de la séquence artificielle: oligonucléotide EM119
<400> 73
   gatccttcat taacgttaac tgttggtgtg aaacctgata gg 42
<210> 74
   <211> 11
   <212> PRT
   <213> Séquence artificielle
<220>
   <223> Description de la séquence artificielle: peptide muté (oligonucléotides EM119 et EM120)
<400> 74
   Ser Phe Ile Asn Val Asn Cys Trp Cys Glu Thr
   1 5 10
<210> 75
   <211> 42
   <212> ADN
   <213> Séquence artificielle
<220>
   <223> Description de la séquence artificielle: oligonucléotide EM120
<400> 75
   tcgacctatc aggtttcaca ccaacagtta acgttaatga ag 42
<210> 76
   <211> 42
   <212> ADN
   <213> Séquence artificielle
<220>
   <223> Description de la séquence artificielle: oligonucléotide EM127
<400> 76
   gatccttctt gaacattaac tgttggtgtg aaacctgata gg 42
<210> 77
   <211> 11
   <212> PRT
   <213> Séquence artificielle
<220>
   <223> Description de la séquence artificielle: peptide muté (oligonucléotides EM127 et EM128)
<400> 77
   Ser Phe Leu Asn Val Asn Cys Trp Cys Glu Thr
   1 5 10
<210> 78
   <211> 42
   <212> ADN
   <213> Séquence artificielle
<220>
   <223> Description de la séquence artificielle: oligonucléotide EM128
<400> 78
   tcgacctatc aggtttcaca ccaacagtta atgttcaaga ag 42
<210> 79
   <211> 42
   <212> ADN
   <213> Séquence artificielle
<220>
   <223> Description de la séquence artificielle: oligonucléotide EM117
<400> 79
   gatccttcgc taacgttaac tgttggtgtc aaacctgata gg 42
<210> 80
   <211> 11
   <212> PRT
   <213> Séquence artificielle
<220>
   <223> Description de la séquence artificielle: peptide muté (oligonucléotides EM117 et EM 118)
<400> 80
   Ser Phe Ala Asn Val Asn Cys Trp Cys Gln Thr
   1 5 10
<210> 81
   <211> 42
   <212> ADN
   <213> Séquence artificielle
<220>
   <223> Description de la séquence artificielle: oligonucléotide EM118
<400> 81
   tcgacctatc aggtttgaca ccaacagtta acgttagcga ag 42
<210> 82
   <211> 42
   <212> ADN
   <213> Séquence artificielle
<220>
   <223> Description de la séquence artificielle:oligonucléotide EM129
<400> 82
   gatccttctt gaacgttaac tgttggtgtg aaacctgata gg 42
<210> 83
   <211> 11
   <212> PRT
   <213> Séquence artificielle
<220>
   <223> Description de la séquence artificielle: peptide muté (oligonucléotides EM129 et EM130)
<400> 83
   Ser Phe Leu Asn Val Asn Cys Trp Cys Glu Thr
   1 5 10
<210> 84
   <211> 42
   <212> ADN
   <213> Séquence artificielle
<220>
   <223> Description de la séquence artificielle: oligonucléotide 130
<400> 84
   tcgacctatc aggtttcaca ccaacagtta acgttcaaga ag 42
<210> 85
   <211> 42
   <212> ADN
   <213> Séquence artificielle
<220>
   <223> Description de la séquence artificielle: oligonucléotide EM135
<400> 85
   gatccttcgc taacgttaac tgttggtgtg aaagatgata gg 42
<210> 86
   <211> 11
   <212> PRT
   <213> Séquence artificielle
<220>
   <223> Description de la séquence artificielle: peptide muté (oligonucléotides EM135 et EM136)
<400> 86
   Ser Phe Ala Asn Val Asn Cys Trp Cys Glu Arg
   1 5 10
<210> 87
   <211> 42
   <212> ADN
   <213> Séquence artificielle
<220>
   <223> Description de la séquence artificielle: oligonucléotide EM136
<400> 87
   tcgacctatc atctttcaca ccaacagtta acgttagcga ag 42
<210> 88
   <211> 42
   <212> ADN
   <213> Séquence artificielle
<220>
   <223> Description de la séquence artificielle: oligonucléotide EM158
<400> 88
   gatccttctt gaacgttaac tgttggtgtc aaacctgata gg 42
<210> 89
   <211> 11
   <212> PRT
   <213> Séquence artificielle
<220>
   <223> Description de la séquence artificielle: peptide muté (oligonuclméotides EM158 et EM159)
<400> 89
   Ser Phe Leu Asn Val Asn Cys Trp Cys Gln Thr
   1 5 10
<210> 90
   <211> 42
   <212> ADN
   <213> Séquence artificielle
<220>
   <223> Description de la séquence artificielle: oligonucléotide EM159
<400> 90
   tcgacctatc aggtttgaca ccaacagtta acgttcaaga ag 42
<210> 91
   <211> 42
   <212> ADN
   <213> Séquence artificielle
<220>
   <223> Description de la séquence artificielle: oligonucléotide EM162
<400> 91
   gatccttctt gaacgttaac tgttggtgtg aaagatgata gg 42
<210> 92
   <211> 11
   <212> PRT
   <213> Séquence artificielle
<220>
   <223> Description de la séquence artificielle :peptide muté (oligonucléotides EM162 et EM163)
<400> 92
   Ser Phe Leu Asn Val Asn Cys Trp Cys Glu Arg
   1 5 10
<210> 93
   <211> 42
   <212> ADN
   <213> Séquence artificielle
<220>
   <223> Description de la séquence artificielle: oligonucléotide EM163
<400> 93
   tcgacctatc atctttcaca ccaacagtta acgttcaaga ag 42
<210> 94
   <211> 22
   <212> ADN
   <213> Séquence artificielle
<220>
   <223> Description de la séquence artificielle: oligonucléotide EM53
<400> 94
   cctggcaatt ccttaccttc ca 22
<210> 95
   <211> 39
   <212> ADN
   <213> Séquence artificielle
<220>
   <223> Description de la séquence artificielle: oligonucléotide EM137
<400> 95
   ttttttaagc ttggataaaa gaaacaagtt gattggcag 39
<210> 96
   <211> 10
   <212> PRT
   <213> Séquence artificielle
<220>
   <223> Description de la séquence artificielle: peptide muté (oligonucléotides EM53 et EM137)
<400> 96
   Ser Leu Asp Lys Arg Asn Lys Leu Ile Gly
   1 5 10